# FASCICULE DE BREVET EUROPEEN

(11) **EP 2 689 018 B1**
(45) Date de publication et mention de la délivrance du brevet: **13.02.2019**
(21) Numéro de dépôt: 12710105.3
(22) Date de dépôt: 23.03.2012
(51) Int. Cl.: C12N 15/79, C12Q 1/25, C07K 14/21, C07K 19/00, C12N 15/82

(54) **CONSTRUCTIONS ET PROCEDE POUR REGULER L'EXPRESSION DES GENES OU POUR DETECTER ET CONTROLER UN LOCUS D'ADN CHEZ LES EUCARYOTES**
KONSTRUKTE UND VERFAHREN ZUR REGULIERUNG DER GENEXPRESSION ODER FÜR DEN NACHWEIS UND DIE KONTROLLE EINER DNA-STELLE BEI EUKARYOTEN
CONSTRUCTS AND METHOD FOR REGULATING GENE EXPRESSION OR FOR DETECTING AND CONTROLLING A DNA LOCUS IN EUKARYOTES

(30) Priorité: 24.03.2011 FR 1152473
(43) Date de publication de la demande: 29.01.2014
(73) Titulaire: Centre National de la Recherche Scientifique, 75016 Paris (FR)
(72) Inventeur: BYSTRICKY, Kerstin, F-31320 Vigoulet-Auzil (FR); GALLARDO, Franck, 82710 Bressols (FR); LANE, David, F-31000 Toulouse (FR); DUBARRY, Nelly, Oxford OX1 1UD (GB)
(74) Mandataire: Ahner, Philippe
(86) Numéro de dépôt international: PCT/EP2012/055258
(87) Numéro de publication internationale: WO 2012/127047

(56) Documents cités:
- J A Surtees ET AL: "P1 ParB Domain Structure Includes Two Independent Multimerization Domains", Journal of Bacteriology, 1 octobre 1999 (1999-10-01), pages 5898-5908, XP055012128, Extrait de l'Internet: URL:http://jb.asm.org/content/181/19/5898. full.pdf+html?sid=6e576f79-6c97-45ae-be28- 68208aac7f72 [extrait le 2011-11-15] cité dans la demande
- YONGFANG LI ET AL: "The P1 plasmid is segregated to daughter cells by a 'capture and ejection' mechanism coordinated with Escherichia coli cell division", MOLECULAR MICROBIOLOGY, vol. 46, no. 1, 1 octobre 2002 (2002-10-01), pages 63-74, XP055012115, ISSN: 0950-382X, DOI: 10.1046/j.1365-2958.2002.03156.x cité dans la demande
- M A DAVIS ET AL: "Recognition of the P1 plasmid centromere analog involves binding of the ParB protein and is modified by a specific host factor", THE EMBO JOURNAL, vol. 7, no. 6, 1 janvier 1988 (1988-01-01) , pages 1881-1888, XP55027253,
- JOFFE B ET AL: "Differentiation and large scale spatial organization of the genome", CURRENT OPINION IN GENETICS & DEVELOPMENT, CURRENT BIOLOGY LTD, XX, vol. 20, no. 5, 1 octobre 2010 (2010-10-01), pages 562-569, XP027288622, ISSN: 0959-437X [extrait le 2010-06-17]

### SEQUENCE LISTING

<110> Centre National de la Recherche Scientifique
<120> Constructions et procédé pour réguler l'expression des gènes ou pour détecter et contrôler un locus d'ADN chez les eucaryotes
<130> SP 39389 (PCT)/SG
<140> PCT/EP2012/XXXXX
   <141> 2012-03-23
<150> FR11/52473
   <151> 2011-03-24
<160> 21
<170> PatentIn version 3.5
<210> 1
   <211> 1062
   <212> DNA
   <213> Burkholderia cenocepacia
<220>
   <221> CDS
   <222> (1)..(1062)
   <223> Séquence codant la protéine ParB portée par le chromosome 2 de Bcc
<400> 1
   atg aaa ccc tcc caa ttt gcc aaa gga ttc caa gcg cgc ccg gat atc        48
   Met Lys Pro Ser Gln Phe Ala Lys Gly Phe Gln Ala Arg Pro Asp Ile
   1               5                   10                  15
   acg acg agc gag aag cgc acg gcg ctt gat cgg ctc aat gcg atc gac        96
   Thr Thr Ser Glu Lys Arg Thr Ala Leu Asp Arg Leu Asn Ala Ile Asp
               20                  25                  30
   ggc atc gtc aag tcc gag acg ccg acc ccg gcg ccg acc aaa tcc gcg       144
   Gly Ile Val Lys Ser Glu Thr Pro Thr Pro Ala Pro Thr Lys Ser Ala
           35                  40                  45
   aag aag gac atc gca ccg ccg cct gct ccg gaa ttc acg atc gat ccg       192
   Lys Lys Asp Ile Ala Pro Pro Pro Ala Pro Glu Phe Thr Ile Asp Pro
       50                  55                  60
   tcg atc gac gaa tcg cag caa tat cgc gcg tgg cgt ctc gag aat cgc       240
   Ser Ile Asp Glu Ser Gln Gln Tyr Arg Ala Trp Arg Leu Glu Asn Arg
   65                  70                  75                  80
   tat gca ccc ggg cag gtg atc gag ctg ccg ctg aag gcg atc aag cac       288
   Tyr Ala Pro Gly Gln Val Ile Glu Leu Pro Leu Lys Ala Ile Lys His
                   85                  90                  95
   agc ccg ttc aac ccg cgg cac ttc tat ctg aaa tcg tcg att gcc gaa       336
   Ser Pro Phe Asn Pro Arg His Phe Tyr Leu Lys Ser Ser Ile Ala Glu
               100                 105                 110
   ctc gcg gtc aac ctc gcg aag cag gga cag cag cag gcg atc cac gtg       384
   Leu Ala Val Asn Leu Ala Lys Gln Gly Gln Gln Gln Ala Ile His Val
           115                 120                 125
   att ccg gat tac gac aac ccg ggc acg tat ttc gtc agc gat ggc ggg        432
   Ile Pro Asp Tyr Asp Asn Pro Gly Thr Tyr Phe Val Ser Asp Gly Gly
       130                 135                 140
   cgt cgc gtg cgg gcg ctg aag gaa gcg aac aag gaa tcg gtc aag gcg        480
   Arg Arg Val Arg Ala Leu Lys Glu Ala Asn Lys Glu Ser Val Lys Ala
   145                 150                 155                 160
   atc gtg atc gat gtg ccg atc ggc atc cag agc tac aag ctc ggc tac        528
   Ile Val Ile Asp Val Pro Ile Gly Ile Gln Ser Tyr Lys Leu Gly Tyr
                   165                 170                 175
   gac ctg aac gtt cag cgc gat tcg cag acg gtg ttc gac aac gcc gtc        576
   Asp Leu Asn Val Gln Arg Asp Ser Gln Thr Val Phe Asp Asn Ala Val
               180                 185                 190
   gtg tgg cgc cgc ttc ctc gac gac aag cat ttc cag agc cag aaa gaa        624
   Val Trp Arg Arg Phe Leu Asp Asp Lys His Phe Gln Ser Gln Lys Glu
           195                 200                 205
   ctc tcc gag cat ctc ggc ctc gac gag tcg acg gtg gcc gtc gcg ctg        672
   Leu Ser Glu His Leu Gly Leu Asp Glu Ser Thr Val Ala Val Ala Leu
       210                 215                 220
   tcg atc ggc aag ttg ccg gaa gcg atc atg cag gaa atg gtc gca cgc        720
   Ser Ile Gly Lys Leu Pro Glu Ala Ile Met Gln Glu Met Val Ala Arg
   225                 230                 235                 240
   ccc gat cgc ttc gga tcg aac atg gcg tat cag gtc ggc cgc tat cac        768
   Pro Asp Arg Phe Gly Ser Asn Met Ala Tyr Gln Val Gly Arg Tyr His
                   245                 250                 255
   aat gcg cgc ggc acc gag gcc acg ctg cgg ctg atc aac aag atc gtg        816
   Asn Ala Arg Gly Thr Glu Ala Thr Leu Arg Leu Ile Asn Lys Ile Val
               260                 265                 270
   tcg gac gat ctc agc acg cgc cag gtg tcg gac atc gtc aag ggt cgc        864
   Ser Asp Asp Leu Ser Thr Arg Gln Val Ser Asp Ile Val Lys Gly Arg
           275                 280                 285
   gtc gcg gca cag gag acg ccg aag cct gcg ggc cgt cag cgc tat gcg        912
   Val Ala Ala Gln Glu Thr Pro Lys Pro Ala Gly Arg Gln Arg Tyr Ala
       290                 295                 300
   cag cgt ctc gag atc aag ctc ggc ggc aag tcg gtc ggc gac ctg aag        960
   Gln Arg Leu Glu Ile Lys Leu Gly Gly Lys Ser Val Gly Asp Leu Lys
   305                 310                 315                 320
   tcg tat ggc gaa gac cgc atc gaa ttg cgc ctg cgc ggc ctc ccg aag       1008
   Ser Tyr Gly Glu Asp Arg Ile Glu Leu Arg Leu Arg Gly Leu Pro Lys
                   325                 330                 335
   gac aag cgc gac gcg att ctc gag cag ctc gag cgg atg ctg ttg tcg       1056
   Asp Lys Arg Asp Ala Ile Leu Glu Gln Leu Glu Arg Met Leu Leu Ser
               340                 345                 350
   gag cat                                                               1062
   Glu His
<210> 2
   <211> 354
   <212> PRT
   <213> Burkholderia cenocepacia
<400> 2
   Met Lys Pro Ser Gln Phe Ala Lys Gly Phe Gln Ala Arg Pro Asp Ile
   1               5                   10                  15
   Thr Thr Ser Glu Lys Arg Thr Ala Leu Asp Arg Leu Asn Ala Ile Asp
               20                  25                  30
   Gly Ile Val Lys Ser Glu Thr Pro Thr Pro Ala Pro Thr Lys Ser Ala
           35                  40                  45
   Lys Lys Asp Ile Ala Pro Pro Pro Ala Pro Glu Phe Thr Ile Asp Pro
       50                  55                  60
   Ser Ile Asp Glu Ser Gln Gln Tyr Arg Ala Trp Arg Leu Glu Asn Arg
   65                  70                  75                  80
   Tyr Ala Pro Gly Gln Val Ile Glu Leu Pro Leu Lys Ala Ile Lys His
                   85                  90                  95
   Ser Pro Phe Asn Pro Arg His Phe Tyr Leu Lys Ser Ser Ile Ala Glu
               100                 105                 110
   Leu Ala Val Asn Leu Ala Lys Gln Gly Gln Gln Gln Ala Ile His Val
           115                 120                 125
   Ile Pro Asp Tyr Asp Asn Pro Gly Thr Tyr Phe Val Ser Asp Gly Gly
       130                 135                 140
   Arg Arg Val Arg Ala Leu Lys Glu Ala Asn Lys Glu Ser Val Lys Ala
   145                 150                 155                 160
   Ile Val Ile Asp Val Pro Ile Gly Ile Gln Ser Tyr Lys Leu Gly Tyr
                   165                 170                 175
   Asp Leu Asn Val Gln Arg Asp Ser Gln Thr Val Phe Asp Asn Ala Val
               180                 185                 190
   Val Trp Arg Arg Phe Leu Asp Asp Lys His Phe Gln Ser Gln Lys Glu
           195                 200                 205
   Leu Ser Glu His Leu Gly Leu Asp Glu Ser Thr Val Ala Val Ala Leu
       210                 215                 220
   Ser Ile Gly Lys Leu Pro Glu Ala Ile Met Gln Glu Met Val Ala Arg
   225 230 235 240
   Pro Asp Arg Phe Gly Ser Asn Met Ala Tyr Gln Val Gly Arg Tyr His
   245 250 255
   Asn Ala Arg Gly Thr Glu Ala Thr Leu Arg Leu Ile Asn Lys Ile Val
   260 265 270
   Ser Asp Asp Leu Ser Thr Arg Gln Val Ser Asp Ile Val Lys Gly Arg
   275 280 285
   Val Ala Ala Gln Glu Thr Pro Lys Pro Ala Gly Arg Gln Arg Tyr Ala
   290 295 300
   Gln Arg Leu Glu Ile Lys Leu Gly Gly Lys Ser Val Gly Asp Leu Lys
   305 310 315 320
   Ser Tyr Gly Glu Asp Arg Ile Glu Leu Arg Leu Arg Gly Leu Pro Lys
   325 330 335
   Asp Lys Arg Asp Ala Ile Leu Glu Gln Leu Glu Arg Met Leu Leu Ser
   340 345 350
   Glu His
<210> 3
   <211> 1032
   <212> DNA
   <213> Burkholderia cenocepacia
<220>
   <221> CDS
   <222> (1)..(1032)
   <223> Séquence codant la protéine ParB portée par le chromosome 3 de Bcc
<400> 3
   atg gct aag gac acc tcg aaa gac aag aaa ccg acc ggc aac ctg cat 48
   Met Ala Lys Asp Thr Ser Lys Asp Lys Lys Pro Thr Gly Asn Leu His
   1 5 10 15
   ctc gcg gcc ggc ctg ttg cgc gga ctc gcg cag gaa aat gcg gcg ctc 96
   Leu Ala Ala Gly Leu Leu Arg Gly Leu Ala Gln Glu Asn Ala Ala Leu
   20 25 30
   gaa acg cgg ctg ccc gag ccg acg gcc gca ccg gct gcg gtc gtc gaa 144
   Glu Thr Arg Leu Pro Glu Pro Thr Ala Ala Pro Ala Ala Val Val Glu
   35 40 45
   gcg acg ccc gcc agt gct ccg gcc gcc gcc acg ccc gcc ggt acg gcc 192
   Ala Thr Pro Ala Ser Ala Pro Ala Ala Ala Thr Pro Ala Gly Thr Ala
   50 55 60
   gat ctt ggc gcg ccg cag aag gtg ctg gtc aag gac tgc atc ccg aac        240
   Asp Leu Gly Ala Pro Gln Lys Val Leu Val Lys Asp Cys Ile Pro Asn
   65                  70                  75                  80
   ccg ttc aat ccg cgt gtg ttc tac tcg gaa tcg agc ctg cac gag ctc        288
   Pro Phe Asn Pro Arg Val Phe Tyr Ser Glu Ser Ser Leu His Glu Leu
                   85                  90                  95
   gca ctg acg ttg aaa cgg gaa ggg cag atc gag ccg atc aag gtc acg        336
   Ala Leu Thr Leu Lys Arg Glu Gly Gln Ile Glu Pro Ile Lys Val Thr
               100                 105                 110
   cga ctc ccc gag ttt ccc ggc aag ctc gtc gtg atc gac ggg caa cgc        384
   Arg Leu Pro Glu Phe Pro Gly Lys Leu Val Val Ile Asp Gly Gln Arg
           115                 120                 125
   cgg ctg cgt gcg acg agc atc aac ggc gac gag acg atc aat gcg acg        432
   Arg Leu Arg Ala Thr Ser Ile Asn Gly Asp Glu Thr Ile Asn Ala Thr
       130                 135                 140
   ttc cgc acg gat cac acg ccc gag cag ctc tat acg atc gcg tat cgc        480
   Phe Arg Thr Asp His Thr Pro Glu Gln Leu Tyr Thr Ile Ala Tyr Arg
   145                 150                 155                 160
   gcg aac cac gac cac gaa cgc cag acg atc ttc gac gat gcg gtt gcg        528
   Ala Asn His Asp His Glu Arg Gln Thr Ile Phe Asp Asp Ala Val Ala
                   165                 170                 175
   tgg aaa cgt ctc ctc gac gag aag gtc ttt tcc gac cag aac acg ctg        576
   Trp Lys Arg Leu Leu Asp Glu Lys Val Phe Ser Asp Gln Asn Thr Leu
               180                 185                 190
   gcg gaa aaa atc ggc aag gac aag gcc tcg atc agc aag acg ctg tcg        624
   Ala Glu Lys Ile Gly Lys Asp Lys Ala Ser Ile Ser Lys Thr Leu Ser
           195                 200                 205
   ctc aac gcg ctg ccg aac acg ctg ctg gag cgg atg gcc agt gcg aac        672
   Leu Asn Ala Leu Pro Asn Thr Leu Leu Glu Arg Met Ala Ser Ala Asn
       210                 215                 220
   gac gtc gtc ggt ctg cag gcg gcg tac ttc ctc aaa ctg atc tat gaa        720
   Asp Val Val Gly Leu Gln Ala Ala Tyr Phe Leu Lys Leu Ile Tyr Glu
   225                 230                 235                 240
   cgc ctg ggc gag ccg acg gcc gac cgg ctg ctg acg gcc gtg atc gac        768
   Arg Leu Gly Glu Pro Thr Ala Asp Arg Leu Leu Thr Ala Val Ile Asp
                   245                 250                 255
   cgg aaa aaa tcc gtc cgc gat ctc gag aat ttc ctg cgt gcg cag agc        816
   Arg Lys Lys Ser Val Arg Asp Leu Glu Asn Phe Leu Arg Ala Gln Ser
               260                 265                 270
   gac ggc acc aag aag gcc gga cgc acg cgc tac agc gtt cgt cac gac        864
   Asp Gly Thr Lys Lys Ala Gly Arg Thr Arg Tyr Ser Val Arg His Asp
           275                 280                 285
   ttc gcg ctc gaa tcg cgc gcg atc ggc cag ttg aag acg tat ccg gac        912
   Phe Ala Leu Glu Ser Arg Ala Ile Gly Gln Leu Lys Thr Tyr Pro Asp
       290                 295                 300
   ggg cgt ctg gat ctg caa ctc aag ggc gtc gac gca tcc cac cag gaa        960
   Gly Arg Leu Asp Leu Gln Leu Lys Gly Val Asp Ala Ser His Gln Glu
   305                 310                 315                 320
   gcg ctt gcc gac aag ctc aag acc gtc atc gac gcg tac gtg gcg gat           1008
   Ala Leu Ala Asp Lys Leu Lys Thr Val Ile Asp Ala Tyr Val Ala Asp
                   325                 330                 335
   ctg gcg acg gcc acg tca aag cat                                           1032
   Leu Ala Thr Ala Thr Ser Lys His
               340
<210> 4
   <211> 344
   <212> PRT
   <213> Burkholderia cenocepacia
<400> 4
   Met Ala Lys Asp Thr Ser Lys Asp Lys Lys Pro Thr Gly Asn Leu His
   1               5                   10                  15
   Leu Ala Ala Gly Leu Leu Arg Gly Leu Ala Gln Glu Asn Ala Ala Leu
               20                  25                  30
   Glu Thr Arg Leu Pro Glu Pro Thr Ala Ala Pro Ala Ala Val Val Glu
           35                  40                  45
   Ala Thr Pro Ala Ser Ala Pro Ala Ala Ala Thr Pro Ala Gly Thr Ala
       50                  55                  60
   Asp Leu Gly Ala Pro Gln Lys Val Leu Val Lys Asp Cys Ile Pro Asn
   65                  70                  75                  80
   Pro Phe Asn Pro Arg Val Phe Tyr Ser Glu Ser Ser Leu His Glu Leu
                   85                  90                  95
   Ala Leu Thr Leu Lys Arg Glu Gly Gln Ile Glu Pro Ile Lys Val Thr
               100                 105                 110
   Arg Leu Pro Glu Phe Pro Gly Lys Leu Val Val Ile Asp Gly Gln Arg
           115                 120                 125
   Arg Leu Arg Ala Thr Ser Ile Asn Gly Asp Glu Thr Ile Asn Ala Thr
       130                 135                 140
   Phe Arg Thr Asp His Thr Pro Glu Gln Leu Tyr Thr Ile Ala Tyr Arg
   145                 150                 155                 160
   Ala Asn His Asp His Glu Arg Gln Thr Ile Phe Asp Asp Ala Val Ala
                   165                 170                 175
   Trp Lys Arg Leu Leu Asp Glu Lys Val Phe Ser Asp Gln Asn Thr Leu
               180                 185                 190
   Ala Glu Lys Ile Gly Lys Asp Lys Ala Ser Ile Ser Lys Thr Leu Ser
           195                 200                 205
   Leu Asn Ala Leu Pro Asn Thr Leu Leu Glu Arg Met Ala Ser Ala Asn
       210                 215                 220
   Asp Val Val Gly Leu Gln Ala Ala Tyr Phe Leu Lys Leu Ile Tyr Glu
   225                 230                 235                 240
   Arg Leu Gly Glu Pro Thr Ala Asp Arg Leu Leu Thr Ala Val Ile Asp
                   245                 250                 255
   Arg Lys Lys Ser Val Arg Asp Leu Glu Asn Phe Leu Arg Ala Gln Ser
               260                 265                 270
   Asp Gly Thr Lys Lys Ala Gly Arg Thr Arg Tyr Ser Val Arg His Asp
           275                 280                 285
   Phe Ala Leu Glu Ser Arg Ala Ile Gly Gln Leu Lys Thr Tyr Pro Asp
       290                 295                 300
   Gly Arg Leu Asp Leu Gln Leu Lys Gly Val Asp Ala Ser His Gln Glu
   305                 310                 315                 320
   Ala Leu Ala Asp Lys Leu Lys Thr Val Ile Asp Ala Tyr Val Ala Asp
                   325                 330                 335
   Leu Ala Thr Ala Thr Ser Lys His
               340
<210> 5
   <211> 16
   <212> DNA
   <213> unknown
<220>
   <223> Site de reconnaissance par la protéine liant l'ADN et appartenant au système de partition de l'ADN bactérien
<220>
   <221> misc_feature
   <222> (1)..(2)
   <223> n représente a, g, c ou t
<220>
   <221> misc_feature
   <222> (4)..(7)
   <223> n représente a, g, c ou t
<220>
   <221> misc_feature
   <222> (10)..(13)
   <223> n représente a, g, c ou t
<220>
   <221> misc_feature
   <222> (15)..(16)
   <223> n représente a, g, c ou t
<400> 5
   nntnnnncgn nnnann 16
<210> 6
   <211> 16
   <212> DNA
   <213> unknown
<220>
   <223> Site de reconnaissance par la protéine liant l'ADN et appartenant au système de partition de l'ADN bactérien
<220>
   <221> misc_feature
   <222> (1)..(1)
   <223> n représente g, c ou t
<220>
   <221> misc_feature
   <222> (4)..(4)
   <223> n représente t ou g
<220>
   <221> misc_feature
   <222> (5)..(5)
   <223> n représente a ou t
<220>
   <221> misc_feature
   <222> (6)..(6)
   <223> n représente t ou c
<220>
   <221> misc_feature
   <222> (7)..(7)
   <223> n représente g ou a
<220>
   <221> misc_feature
   <222> (10)..(10)
   <223> n représente t ou c
<220>
   <221> misc_feature
   <222> (11)..(11)
   <223> n représente g ou a
<220>
   <221> misc_feature
   <222> (12)..(12)
   <223> n représente a ou t
<220>
   <221> misc_feature
   <222> (13)..(13)
   <223> n représente a ou c
<400> 6
   nttnnnncgn nnnaac 16
<210> 7
   <211> 16
   <212> DNA
   <213> Burkholderia cenocepacia
<220>
   <221> protein_bind
   <222> (1)..(16)
   <223> Site de reconnaissance par ParB au niveau de ParS-c2
<400> 7
   gtttatgcgc ataaac 16
<210> 8
   <211> 16
   <212> DNA
   <213> Burkholderia cenocepacia
<220>
   <221> protein_bind
   <222> (1)..(16)
   <223> Site de reconnaissance par ParB au niveau de ParS-c2
<400> 8
   ctttatgcgc ataaac 16
<210> 9
   <211> 16
   <212> DNA
   <213> Burkholderia cenocepacia
<220>
   <221> protein_bind
   <222> (1)..(16)
   <223> Site de reconnaissance par ParB au niveau de ParS-c3
<400> 9
   gttgtcacgt gacaac 16
<210> 10
   <211> 16
   <212> DNA
   <213> Burkholderia cenocepacia
<220>
   <221> protein_bind
   <222> (1)..(10)
   <223> Site de reconnaissance par ParB au niveau de ParS-c3
<400> 10
   tttgtcacgt gacaac 16
<210> 11
   <211> 16
   <212> DNA
   <213> Burkholderia cenocepacia
<220>
   <221> protein_bind
   <222> (1)..(16)
   <223> Site de reconnaissance par ParB au niveau de ParS-c3
<400> 11
   cttgtcacgt gacaac 16
<210> 12
   <211> 1049
   <212> DNA
   <213> Burkholderia cenocepacia
<220>
   <221> gene
   <222> (1)..(1049)
   <223> Séquence ParS portée par le chromosome 2 de Bcc
<400> 12
   cgtacgctgc aggtcgacgg atccccccat tcggacgatc ggtttatgcg cataaacgag 60
   cggtggatgt tcgatggctc gtttggacgg tgtggtgaag aggaagcggc tgatgccgag 120
   tgatcgatgc cctgctcagg attacgtctt catgggcgta tcgtcggggc cgttcgtggc 180
   agagatatct cgacgctctc tcctttcgcg ggacgagccg cgttgcctgc gagccgcgag 240
   tttgtgcgca taaagactgc ggaggccgat cgatgggctt cgtcgctaga gcgcatggta 300
   ttcactcgcg agcgtccggc gtgagctgcg tgtcgtagtc aggtgcgtag atcacgaggt 360
   gcgtcgttga atggacgtgc atggaggcaa tgtcctgccg ggccgtagtg tttatgcgca 420
   taaaccagag gcaacgatag gccgcgacga agcgccgttg acagagacta cgcctgagtc 480
   aggcgggcaa tgacgtcatg tcggggacga cggtctatcg atatagccat cgcgcagtgc 540
   cgttgcgtgt cgacatatgt ccatgccacg agccacgctg tacctcgccc ggcaatcgcg 600
   cgctttatgc gcataaacct agccggcgtg ttccgcaatc gcatacccct ttcgcgaccc 660
   gagacagccc tcacgagaca gccctcacga gacagccctc acgagacagc cctcacgaga 720
   catcggctgc gccggtcgtc ggccccgcga agccacccgc ccgactttat gcgcataaac 780
   aaccactctt cgacgcgtcg ccgaacacag gacactcccc tctcgcgatc actgcgcatc 840
   gccaaaccga aaccgcctca gcgacctatc aagaggaacc ccacaccgat ctcgattgcc 900
   ccccatcagg cccctcgatg cagactcgct acaatacagg cctcacccct cctgccttga      960
   acgatgatca cgatctacca caacccccga tgctcgaaat cccgcgagac gcttgcgttg      1020
   gtcgagtcgc tgaataccgc cggcgcgcc                                       1049
<210> 13
   <211> 1181
   <212> DNA
   <213> Burkholderia cenocepacia
<220>
   <221> gene
   <222> (1)..(1181)
   <223> Séquence ParS portée par le chromosome 3 de Bcc
<400> 13
   ccggcgtcaa cttctatcta ctccgaatcc caccattcca tgaatcgcgc gaccgacgtg      60
   tgcgttgtca cgtgacaact ggcgctaaat atcggtgcgc cccgtactcg attggaggct      120
   caagggaatt caatggtaga gcaccgatcc tcccggctca tcggcggatc ccgcgaagaa      180
   tcagccgaaa cgtatcaaaa acaacaaatc acatatcaat caaaacaaat ctagccgcag      240
   ccgtatcaat cttcaatgcc cgggcgttga aaactcatac gcccctattt cccgagggtg      300
   cagcatccgt tttgcgtgaa acagcatgaa gaatatggcg caaaccattg cccgacaagg      360
   atttccgggc gagtgcttat tttcgggaaa tgtttcacga tccgacccgt ttgggttgtt      420
   acatgcctct gacgtatgga ttcagcgcca gtagcagttc atacctgcgg ctatctccgg      480
   catcaacagt ccaccagcgc aggcaccggc tggcggccac gaagcaacct accctatcgg      540
   ccggcacgcg tggaaacgca gggtcgcgct cctataaatg gggacatatc atgacagccc      600
   catgcagcct ttgcgacaga gctcggcagc attgcgtccg cccttgaggg tccgcacgcc      660
   ggcaggaatt tggccgcgca accgatcgtg ccaaggcctt cgcagcccca agccgtttct      720
   ttgcgacagc aacggggcgc tcctcgccat agagtcgcca tcgcttgaac ttcccggcat      780
   cgcgtcctgt tgtcacgtga caactcaccg atggacacac ggacaaacca cctcggcgaa      840
   aactatcaac ttcgtgcgtt tccccgacaa tatgcctcca cgccgcgagc gtcgaccagc      900
   aacaactatt cttaactgac cacaaatccc ctatccgaga cagcaccttg ggattgtttg      960
   tcacgtgaca actcatcttc ccaggtagac gtccgcgatt tagcgcccgt gtatcgataa      1020
   ttagagtgaa gcgattaccc cgcgctccag gtcgcaatcg aaaactttcg ctcgacgtag      1080
   cactcggagg aatcgatatc cggcgacttg tcacgtgaca accggctctt cctcgttacc      1140
   ggggtctcag cgccccggat ttgggttaat taaggcgcgc c                         1181
<210> 14
   <211> 28
   <212> DNA
   <213> unknown
<220>
   <223> Amorce E
<400> 14
   attagcggat cctacctgac gcttttta 28
<210> 15
   <211> 34
   <212> DNA
   <213> unknown
<220>
   <223> Amorce F
<400> 15
   ggaattgcgg ccgcttactt gtacagctcg tcca 34
<210> 16
   <211> 75
   <212> DNA
   <213> unknown
<220>
   <223> Oligonucléotide G
<400> 16
   cttcaaagaa atatttaaac tcatttatgg cttttagagc atattactca gtgacactat 60
   agaacgcggc cgcca 75
<210> 17
   <211> 75
   <212> DNA
   <213> Unknown
<220>
   <223> Oligonucléotide H
<400> 17
   tcagcgagca gagaagacaa gacattttgt tttacaccgg agccaaactg tatagggaga 60
   ccggcagatc cgcgg 75
<210> 18
   <211> 25
   <212> DNA
   <213> unknown
<220>
   <223> Amorce A
<400> 18
   gtgacactat agaacgcggc cgcca 25
<210> 19
   <211> 25
   <212> DNA
   <213> unknown
<220>
   <223> Amorce B
<400> 19
   tatagggaga ccggcagatc cgcgg 25
<210> 20
   <211> 32
   <212> DNA
   <213> unknown
<220>
   <223> Amorce C
<400> 20
   tccagccctc actccttctc taggcgccgg aa 32
<210> 21
   <211> 30
   <212> DNA
   <213> unknown
<220>
   <223> Amorce D
<400> 21
   gttctccaac ttcaagaaac tgttacccat 30

## Description

### DOMAINE TECHNIQUE

La présente invention concerne, de façon générale, le domaine des biotechnologies et notamment le domaine des outils utiles en biotechnologie.

Plus particulièrement, la présente invention propose d'utiliser des séquences issues du système de partition de l'ADN plasmidique et chromosomique des bactéries, dans la régulation de l'expression des gènes dans les cellules eucaryotes et notamment les cellules eucaryotes vivantes et/ou dans la détection et le contrôle de loci d'ADN génomique d'intérêt dans de telles cellules.

La présente invention concerne donc les constructions mises en oeuvre dans ces procédés de régulation, détection et contrôle, telles que vecteurs d'expression eucaryotes, protéines de fusion et polynucléotides les codant ainsi que des cellules eucaryotes transformées par ou exprimant de telles constructions.

La présente invention concerne également un procédé pour réguler l'expression des gènes dans une cellule eucaryote et un procédé pour détecter et contrôler un locus d'ADN génomique d'intérêt dans une cellule eucaryote.

### ÉTAT DE LA TECHNIQUE ANTÉRIEURE

La visualisation de loci d'ADN génomique *in vivo* est typiquement réalisée en utilisant un système à deux composantes impliquant une séquence cible d'ADN (appelée opérateur) qui est reconnue et liée, de façon spécifique, par une protéine (appelée répresseur) fluorescente (Belmont, 2001; Belmont & Straight, 1998; Straight et al, 1996). Ce système, appelé FROS (pour « Fluorescent Repressor Operator System *»),* a été couramment utilisé pour détecter la position de régions d'ADN dans les cellules vivantes des bactéries jusqu'aux cellules de mammifères.

A ce jour, seulement deux systèmes de type Opérateur/Répresseur que sont les systèmes LacO/LacI et TetO/TetR, sont disponibles pour l'imagerie *in vivo* (Bystricky et al, 2005 ; Michaelis et al, 1997 ; Therizols et al, 2010) chez les eucaryotes.

Un des inconvénients de ces systèmes repose sur la taille et la nature répétée des séquences opératrices. En effet, ce type d'opérateur est composé de plus de deux cents répétitions d'une séquence opératrice bactérienne. De ce fait, dans sa version courante, l'opérateur LacO inclut plus de 10 kb d'ADN.

Même si cette technique a permis la visualisation de régions d'ADN *in vivo,* l'insertion de ces énormes séquences peut modifier le comportement de la fibre de chromatine. De plus, l'étirement et la compaction de régions chromosomiques de cette taille peut fausser la mise en place d'études dynamiques comme le suivi des particules fluorescentes au cours du temps.

De ce fait, l'étude dans les cellules vivantes de l'architecture et de la dynamique des chromosomes est grandement limitée par la taille de la séquence opératrice elle-même. Des techniques impliquant la détection de courtes séquences d'ADN non répétées n'ont pas encore été développées pour les eucaryotes.

Il existe donc un réel besoin pour des systèmes et constructions permettant d'étudier, dans les cellules eucaryotes notamment vivantes, l'architecture et la dynamique des chromosomes. Les inventeurs se sont fixé pour but d'identifier de tels systèmes et de telles constructions.

La partition ou ségrégation des chromosomes lors de la mitose chez les bactéries est réalisée par un système à trois éléments : une séquence courte, appelée ParS, une protéine ParB qui se lie à ParS et une deuxième protéine ParA avec une activité ATPase qui mobilise les complexes ParB-ParS (Lin & Grossman, 1998). Sur les chromosomes, ParS est typiquement présent en quelques exemplaires repartis dans la région de l'origine de replication (Livny et al, 2007). ParB se lie spécifiquement au niveau d'une séquence de fixation présente au niveau de la séquence ParS. Une fois fixée, ParB est capable de recruter d'autres copies d'elle-même grâce à son domaine N-terminal. De plus, ParB s'étale sur l'ADN à proximité du site de liaison de façon bidirectionnelle (Murray et al, 2006 ; Lynch & Wang, 1995). La plupart des espèces bactériennes contiennent un seul chromosome et des sites parS de séquence identique ou presque.

Par contraste, la bactérie *Burkholderia cenocepacia* (*Bcc*) contient trois chromosomes et un plasmide à bas nombre de copies qui sont indépendamment partitionnés par des systèmes de partition spécifiques appelés Par-c1 (système de partition du chromosome 1), Par-c2 (système de partition du chromosome 2), Par-c3 (système de partition du chromosome 3) et Par-pl (système de partition du plasmide). Chacun de ces quatre replicons porte quelques copies d'un parS de séquence spécifique réparties sur environ 1 kb (Dubarry et al, 2006). Dans cet article, différentes constructions plasmidiques ont été préparées, comme des plasmides dérivés de pDAG203 dans lesquels la séquence parS issue de l'un quelconque des chromosomes ou du plasmide de *Bcc* a été insérée. De tels plasmides ont été utilisés pour transformer la bactérie *Escherichia coli* (*E. coli*). Toutefois, ces plasmides ne constituent pas des vecteurs eucaryotes tels que définis ci-après.

Le système de partition de l'ADN plasmidique et chromosomique des bactéries et notamment le système basé sur le centromère ParS et la protéine ParB qui s'y fixe n'a été utilisé que chez les bactéries. Plus particulièrement, les travaux connus à ce jour ont porté sur la protéine ParB du plasmide P1 de *E. coli.* Or, cette protéine présente l'inconvénient de ne se fixer sur les sites de fixation présents au niveau de la séquence ParS, qu'en présence d'un facteur de l'hôte appelé IHF (pour « Integration Host Factor ») (Li & Austin, 2002). Cette dépendance vis-à-vis de IHF a grandement limité l'intérêt pour ce système. Les travaux décrits par Li & Austin (2002) utilisent des protéines de fusion correspondant à tout ou partie de la protéine ParB du plasmide P1 de *E. coli,* fusionnée à l'extrémité C-terminale de la protéine à fluorescence verte (ou GFP pour « Green Fluorescent Protein »). Ces protéines de fusion ont été produites en utilisant le plasmide pDSW209 modifié pour comprendre une séquence nucléotidique codant ces protéines de fusion et introduit dans des souches bactériennes telles que W3110 et N100.

De même, dans l'article de Surtees & Funnell (1999), la protéine ParB du plasmide P1 de *E. coli* est utilisée pour détecter et étudier, par la technique du double-hybride, les interactions entre les composants de ParB et notamment leur dimérisation. Dans les constructions réalisées, tout ou partie de la protéine ParB du plasmide P1 de *E. coli* est fusionnée au facteur de transcription eucaryote Gal4. C'est ce facteur qui amène ParB près d'un gène rapporteur permettant la visualisation de l'expression de ce dernier grâce à l'interaction de la protéine ParB fusionnée à Gal4 avec le partenaire que l'on cherche à caractériser. En résumé, l'association à l'ADN de la protéine de fusion telle que décrite dans Surtees & Funnell (1999) est due à Gal4 et non à ParB du plasmide P1 de *E. coli* puisque cette dernière seule est incapable d'une telle association.

### EXPOSÉ DE L'INVENTION

La présente invention définie par les revendications 1 à 18 annexées permet de résoudre les problèmes techniques tels que précédemment définis et d'atteindre le but que se sont fixés les inventeurs.

En effet, les travaux des inventeurs ont permis de mettre au point une méthode générale afin de visualiser et d'étudier la dynamique de loci chromatiniens *in vivo* en dérivant le système de partition de *Bcc* pour son utilisation dans les cellules eucaryotes.

Un des atouts de cette méthode repose sur la taille grandement réduite de l'insertion génomique (1 kb) par rapport aux autres systèmes utilisés (>10 kb) et à la nature faiblement répétée de la séquence de fixation (également désignée, dans la présente, séquence de reconnaissance). De ce fait, toutes les méthodes mises en oeuvre afin de préserver la taille des séquences opératrices de l'état de la technique sont obsolètes dans le système et les procédés selon la présente invention.

De façon remarquable, ces constructions peuvent être utilisées non seulement pour détecter et contrôler des loci génomiques d'intérêt et pour visualiser une étape inconnue du métabolisme de l'ADN telle que la dégradation lors de cassures double brin mais aussi dans la régulation et le contrôle de l'expression des gènes chez les eucaryotes.

Enfin, l'enseignement des travaux des inventeurs utilisant des éléments du système de partition de l'ADN de la bactérie *Bcc* est généralisable aux éléments de tout système de partition de l'ADN d'une quelconque bactérie, à condition que, dans cette bactérie, la protéine liant l'ADN et appartenant au système de partition de l'ADN soit capable de lier, au niveau d'un site de reconnaissance, l'ADN sans nécessiter un autre facteur tel qu'un facteur organique et notamment protéique et, avantageusement, soit également capable de recruter d'autres copies d'elle-même.

Le présent fascicule décrit tout d'abord un vecteur d'expression eucaryote comprenant une séquence nucléotidique codant une protéine de fusion particulière.

Par « vecteur d'expression eucaryote », on entend un vecteur adapté pour l'expression, dans une cellule eucaryote, d'au moins un polypeptide codé par une séquence nucléotidique contenue dans ce vecteur. Un tel vecteur est notamment utile pour transformer un organisme eucaryote hôte et exprimer dans ce dernier une protéine de fusion telle que définie ci-après.

Le vecteur d'expression eucaryote décrit dans ce fascicule comprend, en plus de la séquence nucléotidique codant une protéine de fusion, un (ou plusieurs) élément(s) qui permet(tent) l'expression i.e. la transcription et la traduction de cette séquence nucléotidique.

Le vecteur d'expression eucaryote décrit dans ce fascicule est avantageusement choisi parmi un plasmide, un cosmide, un bactériophage et un virus tel qu'un baculovirus. En particulier, le vecteur de l'invention est un vecteur à réplication autonome comportant des éléments permettant son maintien et sa réplication dans l'organisme hôte comme une origine de réplication. En outre, le vecteur peut comporter des éléments permettant sa sélection dans l'organisme hôte. Ces éléments sont également connus sous le terme de « marqueurs de sélection ». De tels vecteurs d'expression sont bien connus de l'homme du métier et largement décrits dans la littérature.

Un vecteur d'expression eucaryote se distingue d'un vecteur d'expression procaryote par la présence, sur le vecteur eucaryote, d'au moins un élément choisi parmi une origine de réplication de type eucaryote, un marqueur de sélection de type eucaryote, un promoteur de type eucaryote, un amplificateur également connu sous le terme anglais de « enhancer », un signal 3' UTR (pour « UnTranslated Region »), un signal IRES (pour « Internai Ribosome Entry Site ») et un signal de terminaison de la transcription eucaryote comprenant un site de clivage et/ou un signal polyA (pour « signal de polyadénylation »). Le vecteur d'expression eucaryote selon l'invention peut comprendre 2, 3, 4, 5, 6 ou 7 éléments listés ci-dessus et typiquement tous ces éléments.

Par « marqueur de sélection de type eucaryote », on entend un marqueur choisi parmi un gène du métabolisme à utiliser avec un organisme hôte auxotrophe i.e. un gène de sélection qui assure la complémentation avec le gène respectif délété au niveau du génome de l'organisme hôte. Un tel gène peut être le gène *trp-1* à utiliser avec un organisme eucaryote dépourvu de l'enzyme phosphoribosylanthranilate isomérase tel qu'une levure *trp*⁻ ; le gène *URA3* à utiliser avec un organisme eucaryote dépourvu de l'enzyme orotidine 5-phosphate décarboxylase tel qu'une levure ura⁻ ; le gène tk à utiliser avec un organisme eucaryote dépourvu de l'enzyme thymidine kinase ; le gène *ada* à utiliser avec un organisme eucaryote dépourvu de l'enzyme adénosine désaminase ; le gène Apt à utiliser avec un organisme eucaryote dépourvu de l'enzyme adénine phosphoribosyl-transférase ou le gène *Hprt* à utiliser avec un organisme eucaryote dépourvu de l'enzyme Hypoxanthine-guanine phosphoribosyl-transférase. Il convient de souligner que le vecteur d'expression eucaryote selon la présente invention peut également contenir un marqueur de sélection utilisable chez les procaryotes ou chez les eucaryotes tel qu'un gène bactérien de résistance à un antibiotique comme l'ampicilline, la néomycine, l'hygromycine, la généticine, la carboxine, la nourséothricine ou G418.

Par « promoteur de type eucaryote », on entend, dans le cadre de la présente invention, aussi bien un promoteur, constitutif ou inductible, adapté pour toute cellule eucaryote qu'un promoteur, constitutif ou inductible, spécifique d'un tissu particulier. Un promoteur adapté pour toute cellule eucaryote utilisable dans le cadre de la présente invention est notamment choisi parmi le promoteur CMV (pour « CytoMégaloVirus ») et notamment l'intron A de ce promoteur ; le promoteur CYC1-TetO-7 (cf. partie expérimentale pour plus de détails) ; le promoteur précoce SV40 (pour « Simian Virus 40 »), le promoteur HSV (pour « Herpes Simplex Virus ») et le promoteur TEV (pour « Tobacco Etch Virus »). Un promoteur spécifique d'un tissu particulier utilisable dans le cadre de la présente invention est typiquement choisi parmi le promoteur PEPCK (pour « PhosphoEnolPyruvate CarboxyKinase ») spécifique des hépatocytes, le promoteur SPA (pour « Surfactant proteinA ») spécifique des cellules épithéliales notamment pulmonaires, le promoteur MLC1:3 (pour « Myosin light chain ») spécifique des myobastes, le promoteur CEA (pour « CarcinoEmbryonic Antigen ») spécifique des cellules tumorales et le promoteur MCK (pour « Muscle Creatine Kinase ») spécifique des cellules de muscle squelettique. De plus, un promoteur inductible de levure utilisable dans le cadre de la présente invention peut être le promoteur GAL1 inductible par le galactose, le promoteur AOX1 inductible par le méthanol, le promoteur ADH-2 inductible par déplétion en glucose, le promoteur MET15 inductible par déplétion en méthionine ou le promoteur CUP1 inductible par les ions cuivre. Dans le vecteur d'expression eucaryote objet de la présente invention, le promoteur peut être associé à une ou plusieurs séquences régulatrices transcriptionnelles que sont les amplificateurs.

Avantageusement, le vecteur d'expression eucaryote décrit dans ce fascicule comprend, liés entre eux de façon opérationnelle, un promoteur de type eucaryote, une séquence nucléotidique codant une protéine de fusion et un signal de terminaison de la transcription eucaryote comprenant un site de clivage et/ou un signal polyA. On entend par « liés entre eux de façon opérationnelle » selon l'invention, des éléments liés entre eux de façon à ce que le fonctionnement d'un des éléments soit affecté par celui d'un autre. A titre d'exemple, un promoteur est lié de façon opérationnelle à une séquence codante lorsqu'il est capable d'affecter l'expression de cette dernière. Les éléments régulateurs de la transcription, de la traduction et de la maturation des peptides que le vecteur peut comprendre sont connus de l'homme du métier et ce dernier est capable de les choisir en fonction de l'organisme eucaryote hôte dans lequel l'expression ou le clonage doivent être réalisés.

Par « protéine de fusion », on entend une protéine comprenant au moins deux polypeptides d'une même source ou de sources différentes, liés l'un à l'autre de façon fonctionnelle moyennant quoi chaque polypeptide de la protéine de fusion conserve sa fonction ou son activité propre.

Les deux polypeptides de la protéine de fusion peuvent être liés, l'un à l'autre, de façon directe, au moyen d'une liaison peptidique ou, de façon indirecte, un bras espaceur (ou bras de liaison ou agent de jonction) séparant les deux polypeptides. Le 2^{nd} polypeptide peut être, directement ou indirectement, lié à l'extrémité C-terminale ou N-terminale du 1^{er} polypeptide. Dans le cas d'une liaison directe, les séquences nucléotidiques codant chacun des polypeptides sont liées l'une à l'autre dans une orientation 5'-3' moyennant quoi le cadre de traduction des polypeptides codés n'est pas alteré.

Dans le vecteur d'expression eucaryote décrit dans ce fascicule, la séquence nucléotidique code une protéine fusion comprenant un 1^{er} polypeptide correspondant à une protéine liant l'ADN et appartenant au système de partition de l'ADN bactérien, un dérivé ou un fragment de celle-ci. La présente invention concerne donc un vecteur d'expression eucaryote comprenant une séquence nucléotidique codant une protéine de fusion comprenant un 1^{er} polypeptide correspondant à une protéine liant l'ADN et appartenant au système de partition de l'ADN bactérien, un dérivé ou un fragment de celle-ci. Plus particulièrement, la présente invention concerne un vecteur d'expression eucaryote comprenant une séquence nucléotidique codant une protéine de fusion comprenant un 1^{er} polypeptide correspondant à une protéine liant l'ADN, sans qu'un quelconque autre facteur ne soit nécessaire à cette liaison, et appartenant au système de partition de l'ADN bactérien, un dérivé ou un fragment de celle-ci. « Sans qu'un quelconque autre facteur ne soit nécessaire à cette liaison » signifie que la liaison à l'ADN ne dépend que de la protéine liant l'ADN et appartenant au système de partition de l'ADN bactérien mise en oeuvre et du site de reconnaissance au niveau de l'ADN, aucun autre facteur notamment organique et, en particulier, protéique n'étant impliqué dans cette liaison.

Par « système de partition de l'ADN bactérien », on entend le système impliqué dans la partition (ou la ségrégation) du (ou des) chromosome(s) et éventuellement du (ou des) plasmide(s) lors de la mitose chez les bactéries. Ce système est connu dans la littérature sous l'appellation « système *par* ».

Comme précédemment explicité, le système de partition de l'ADN chez les bactéries est un système à 3 éléments comprenant (i) une séquence d'ADN connue, dans la littérature, sous le terme de ParS ou de séquence centromérique ; (ii) une protéine se liant spécifiquement à cette séquence d'ADN et connue, dans la littérature, sous le terme de ParB et (iii) une protéine à activité ATPase connue, dans la littérature, sous le terme de ParA. Ainsi, par « protéine liant l'ADN et appartenant au système de partition de l'ADN bactérien », on entend la protéine ParB. Il convient toutefois de remarquer que la protéine liant l'ADN et appartenant au système de partition de l'ADN bactérien peut également être appelée la « protéine liant une séquence de type centromère » (i.e. une séquence remplissant le rôle de centromère) ou par tout autre nom, comme, par exemple, chez *Bacillus subtilis,* où cette protéine est appelée Spo0J.

Toute protéine liant l'ADN et appartenant au système de partition de l'ADN bactérien est potentiellement utilisable dans le cadre de la présente invention tant que cette liaison ne dépend pas d'un autre facteur tel que la protéine IHF ou un facteur de transcription comme Gal4. De cette façon, dans la protéine de fusion mise en oeuvre dans le cadre de la présente invention, la liaison à l'ADN dépend uniquement du 1^{er} polypeptide correspondant à une protéine liant l'ADN et appartenant au système de partition de l'ADN bactérien, un dérivé ou un fragment de celle-ci. Avantageusement, la protéine liant l'ADN et appartenant au système de partition de l'ADN bactérien est capable, une fois fixée à l'ADN, de recruter d'autres copies d'elle-même et de s'étaler sur l'ADN. L'homme du métier est capable de vérifier, par un travail de routine, si une protéine liant l'ADN et appartenant au système de partition de l'ADN bactérien est utilisable dans le cadre de la présente invention. A cet effet, l'homme du métier peut utiliser plusieurs tests dont par exemple la formation de foci fluorescents ou le gel retard (EMSA). EMSA est un test dans lequel une électrophorèse sur gel de la séquence d'ADN reconnue par la protéine liant l'ADN et appartenant au système de partition de l'ADN bactérien est réalisée, en présence ou en absence de la protéine recombinante. Si, en présence de la protéine, l'ADN migre moins loin qu'en absence de la protéine, cette dernière est capable de se lier seule à l'ADN et est donc incluse dans la portée de la présente invention. Le recrutement d'autres copies de la protéine liant l'ADN peut être visualisé en mettant en contact une séquence d'ADN présentant un site de reconnaissance avec plusieurs protéines liant l'ADN et appartenant au système de partition de l'ADN bactérien, marquées et notamment fluorescente. Le recrutement se matérialise par un focus fluorescent au niveau de l'ADN.

Les protéines liant l'ADN et appartenant au système de partition de l'ADN bactérien, leur séquence en acides aminés et/ou les séquences nucléotidiques les codant sont accessibles dans les bases de données de séquences d'acides aminés ou nucléotidiques telles que Genbank ou NCBI genome project pour les bactéries dont le génome a été séquencé en totalité ou en partie. Si nécessaire, l'homme du métier pourra utiliser des séquences de protéines ParB déjà décrites pour identifier l'analogue de ces dernières dans une bactérie dont le génome n'aurait pas été complétement séquencé ou pour laquelle la protéine liant l'ADN et appartenant au système de partition de l'ADN bactérien ne serait pas encore connue.

Avantageusement, la protéine liant l'ADN et appartenant au système de partition de l'ADN bactérien est une protéine ParB issue de *Burkholderia cenocepacia.* Plus particulièrement, cette protéine est choisie entre :
- la protéine ParB codée par le chromosome 1 de *Bcc* désignée par « ParB-c1 » telle que la protéine de la souche J2315 de *Bcc* accessible dans NCBI genome project sous le N° YP-002229191 ;
- la protéine ParB codée par le chromosome 2 de *Bcc* désignée par « ParB-c2 » telle que la protéine de la souche J2315 de *Bcc* accessible dans NCBI genome project sous le N° YP-002232636 et correspondant à la protéine SEQ ID NO: 2 dans le listage de séquences en annexe ;
- la protéine ParB codée par le chromosome 3 de *Bcc* désignée par « ParB-c3 » telle que la protéine de la souche J2315 de *Bcc* accessible dans NCBI genome project sous le N° YP-002153395 et correspondant à la protéine SEQ ID NO: 4 dans le listage de séquences en annexe ; et
- la protéine ParB codée par le plasmide de *Bcc* désignée par « ParB-pl » telle que la protéine du plasmide pBCJ2315 de la souche J2315 de *Bcc* accessible dans NCBI genome project sous le N° YP-002235439,
un dérivé ou un fragment de celles-ci.

Par « dérivé d'une protéine liant l'ADN et appartenant au système de partition de l'ADN bactérien », on entend des peptides qui présentent au moins 30%, au moins 40%, au moins 45%, au moins 50%, au moins 55%, au moins 60%, au moins 65%, au moins 70%, au moins 75%, au moins 80%, au moins 85%, au moins 90%, au moins 95% et/ou au moins 99% d'identité respectivement avec la séquence d'une protéine liant l'ADN et appartenant au système de partition de l'ADN bactérien et notamment avec les séquences données ci-dessus. Cette définition de dérivé couvre, par conséquent, les homologues des protéines liant l'ADN et appartenant au système de partition de l'ADN bactérien et notamment des protéines ParB de *Bcc* et, en particulier, les homologues de ParB-cl, ParB-c2, ParB-c3 et ParB-pl de la souche J2315. Par « homologue d'une protéine ParB de *Bcc* », on entend aussi bien une forme différente ou équivalente d'une protéine ParB de *Bcc* de souche J2315, isolée d'une souche ou d'une espèce différente de *Burkholderia cenocepacia.*

Par « pourcentage d'identité » entre deux séquences d'acides aminés (ou entre deux séquences nucléotidiques comme envisagé ci-après), on entend, dans le cadre de la présente invention, un pourcentage de résidus d'acides aminés (ou de nucléotides) identiques entre les deux séquences comparées, ce pourcentage étant obtenu après mise en oeuvre du meilleur alignement (alignement optimum) entre les deux séquences. L'homme du métier connaît différentes techniques permettant d'obtenir un tel pourcentage d'identité et impliquant des algorithmes d'homologie ou des programmes d'ordinateur tels que le programme BLAST.

Le pourcentage d'identité est statistique et les différences entre les deux séquences sont distribuées le long de ces séquences. Les différences entre les deux séquences peuvent consister en différents types de modifications des séquences : des délétions, des substitutions ou des additions de résidus d'acides aminés (ou de nucléotides).

De plus, lorsque le pourcentage d'identité de séquences est utilisé en référence à des protéines, on sait que des résidus qui ne sont pas identiques diffèrent souvent par des substitutions d'acides aminés conservatives, dans lesquelles les acides aminés sont substitués par d'autres acides aminés présentant des propriétés chimiques similaires, telles que charge, caractère hydrophile ou hydrophobe, ce qui, par conséquent, ne change pas les propriétés fonctionnelles de la molécule. Lorsque les séquences diffèrent par des substitutions conservatives, le pourcentage d'identité de séquences peut être ajusté à la hausse pour tenir compte de la nature conservative des substitutions. Les séquences qui diffèrent par de telles substitutions conservatives sont dites présenter une "similarité de séquences" ou une "similarité". Les moyens pour réaliser un tel ajustement sont bien connus de l'homme du métier. Typiquement, cela consiste à compter une substitution conservative comme un mésappariement partiel, plutôt que comme un mésappariement total, augmentant de fait le pourcentage d'identité de séquences.

Des tableaux de substitutions conservatives listant des acides aminés similaires d'un point de vue fonctionnel sont bien connus dans l'état de la technique. Ainsi, les 8 groupes suivants contiennent chacun des acides aminés qui sont des substitutions conservatives les uns pour les autres: **(1)** Alanine (A), Glycine (G) ; **(2)** Acide aspartique (D), Acide glutamique (E) ; **(3)** Asparagine (N), Glutamine (Q) ; **(4)** Arginine (R), Lysine (K) ; **(5)** Isoleucine (I), Leucine (L), Méthionine (M), Valine (V) ; **(6)** Phénylalanine (F), Tyrosine (Y), Tryptophane (W) ; **(7)** Sérine (S), Thréonine (T) ; et **(8)** Cystéine (C), Méthionine (M).

Dans le cadre de la présente invention, un dérivé d'une protéine liant l'ADN et appartenant au système de partition de l'ADN bactérien est un polypeptide présentant une similarité dans la séquence d'acides aminés d'au moins 40%, d'au moins 50%, d'au moins 60%, d'au moins 70%, d'au moins 80%, d'au moins 90% ou d'au moins 95% avec la séquence d'acides aminés d'une protéine liant l'ADN et appartenant au système de partition de l'ADN bactérien.

Ainsi, les modifications entre une protéine liant l'ADN et appartenant au système de partition de l'ADN bactérien et un dérivé de celle-ci et notamment entre les protéines ParB de *Bcc* et leurs dérivés peuvent consister en des substitutions, additions ou délétions d'acides aminés. Quel que soit le type de modifications mis en oeuvre, ces dernières n'abolissent pas la capacité d'un dérivé d'une protéine liant l'ADN et appartenant au système de partition de l'ADN bactérien et notamment d'une protéine ParB de *Bcc* à spécifiquement se lier à l'ADN, sans qu'un quelconque autre facteur ne soit nécessaire à cette liaison. Les substitutions envisagées peuvent être des substitutions entre acides aminés équivalents i.e. des acides aminés présentant des homologies structurales ou ne modifiant pas substantiellement les propriétés d'une protéine liant l'ADN et appartenant au système de partition de l'ADN bactérien. En variante, les substitutions envisagées peuvent être des substitutions par des acides aminés non équivalents i.e. des acides aminés ne présentant pas d'homologie structurale. Ces modifications n'abolissent toutefois pas la capacité d'un dérivé d'une protéine liant l'ADN et appartenant au système de partition de l'ADN bactérien et notamment d'une protéine ParB de *Bcc* à spécifiquement se lier à l'ADN.

Les dérivés des protéines liant l'ADN et appartenant au système de partition de l'ADN bactérien et notamment des protéines ParB de *Bcc* peuvent également présenter, par rapport aux séquences des protéines liant l'ADN et appartenant au système de partition de l'ADN bactérien et notamment par rapport aux protéines ParB de *Bcc* et, en particulier, par rapport aux protéines ParB de *Bcc* dont les séquences ont été données ci-dessus, au moins un acide aminé supplémentaire en partie C-terminale et/ou en partie N-terminale, une modification post-traductionelle et/ou une modification chimique en particulier une glycosylation, une amidation, une acylation, une acétylation, une méthylation, ainsi qu'un groupement protecteur qui permet d'éviter leur dégradation. De façon avantageuse, les dérivés peuvent présenter, par rapport aux séquences des protéines liant l'ADN et appartenant au système de partition de l'ADN bactérien et notamment par rapport aux protéines ParB de *Bcc* et, en particulier, par rapport aux protéines ParB de *Bcc* dont les séquences ont été données ci-dessus, 1, 2, 3, 4, 5, 6, 7, 8, 9 ou 10 acide(s) aminé(s) supplémentaire(s) en partie C-terminale et/ou 1, 2, 3, 4, 5, 6, 7, 8, 9 ou 10 acide(s) aminé(s) supplémentaire(s) en partie N-terminale.

Les dérivés des protéines liant l'ADN et appartenant au système de partition de l'ADN bactérien et notamment des protéines ParB de *Bcc* peuvent également être ceux dont un (ou plusieurs) acide(s) aminé(s) est(sont) choisi(s) dans le groupe constitué par des énantiomères, des diastéréoisomères, des acides aminés naturels de conformation D, des bêta acides aminés, des acides aminés alpha substitués, des acides aminés rares notamment l'hydroxyproline, l'hydroxylysine, l'allo-hydroxylysine, la 6-N-méthyllysine, la N-éthylglycine, la N-méthylglycine, la N-éthylasparagine, l'allo-isoleucine, la N-méthylisoleucine, la N-méthylvaline, la pyroglutamine, l'acide aminobutyrique et des acides aminés synthétiques notamment l'ornithine, la norleucine, la norvaline, la cyclohéxyl-alanine et les oméga-acides aminés. Les dérivés des protéines liant l'ADN et appartenant au système de partition de l'ADN bactérien et notamment des protéines ParB de *Bcc* couvrent selon l'invention également les rétropeptides et les rétroinversopeptides, de même que les peptides dont la chaîne latérale d'un dont un (ou plusieurs) acide(s) aminé(s) est substituée par des groupements qui ne modifient pas la capacité des dérivés à se lier spécifiquement à l'ADN.

Ainsi, quel que soit le type de dérivé envisagé, un dérivé d'une protéine liant l'ADN et appartenant au système de partition de l'ADN bactérien et notamment d'une protéine ParB de *Bcc* conserve sa capacité à spécifiquement se lier à l'ADN, ladite liaison ne dépendant d'aucun autre facteur notamment organique et, en particulier, protéique.

Par « fragment d'une protéine liant l'ADN et appartenant au système de partition de l'ADN bactérien », on entend, dans le cadre de la présente invention toute partie ou toute portion d'une protéine liant l'ADN et appartenant au système de partition de l'ADN bactérien et notamment d'une protéine ParB de *Bcc* ayant conservé sa capacité à spécifiquement se lier à l'ADN, ladite liaison ne dépendant d'aucun autre facteur notamment organique et, en particulier, protéique. Un fragment d'une protéine liant l'ADN et appartenant au système de partition de l'ADN bactérien et notamment d'une protéine ParB de *Bcc* présente au moins un acide aminé en moins en partie C-terminale et/ou en partie N-terminale par rapport à la protéine liant l'ADN et appartenant au système de partition de l'ADN bactérien, notamment par rapport à la protéine ParB de *Bcc* et, en particulier, par rapport aux protéines ParB de *Bcc* dont les séquences ont été décrites ci-dessus.

Avantageusement, le fragment d'une protéine liant l'ADN et appartenant au système de partition de l'ADN bactérien et notamment d'une protéine ParB de *Bcc* présente au moins le motif impliqué dans la liaison à l'ADN. Un tel motif correspond au motif de structure hélice-boucle-hélice (ou motif HTH pour « helix-turn-helix ») tel que décrit dans Dubarry et al, 2006. Le motif HTH correspond notamment à la séquence comprise entre les acides aminés 202 et 225 de la séquence SEQ ID NO: 2 dans le listage de séquences en annexe. Il convient toutefois de noter que toute protéine liant l'ADN et appartenant au système de partition de l'ADN bactérien ne présente pas forcément un motif de liaison à l'ADN de structure HTH. En effet, l'homologue de la protéine ParB chez la bactérie TP228 présente un motif de structure « ribbon-helix-helix » à l'extrémité C-terminale (Golovanov et al, 2003).

Il est clair que la protéine de fusion comprenant un 1^{er} polypeptide correspondant à une protéine liant l'ADN et appartenant au système de partition de l'ADN bactérien et notamment une protéine ParB de *Bcc* ou un de ses dérivés ou fragments ne couvre pas une protéine du système de partition bactérien ou un de ses homologues en tant que tel(le).

Dans le vecteur d'expression eucaryote décrit dans ce fascicule, la séquence nucléotidique code une protéine fusion comprenant un 2^{nd} polypeptide qui est soit un polypeptide facilement détectable, soit un polypeptide impliqué dans la régulation de l'expression des gènes.

Par « polypeptide facilement détectable », on entend, dans le cadre de la présente invention, un polypeptide qui peut être détecté par mise en oeuvre d'une technique de détection appropriée avantageusement non invasive telle que la microscopie, la scintigraphie et la fluorescence. Une protéine de fusion comprenant un tel polypeptide facilement détectable permet notamment d'identifier et localiser des sites d'ADN auxquels se lie la protéine liant l'ADN et appartenant au système de partition de l'ADN bactérien et notamment une protéine ParB de *Bcc* qui correspond à l'autre polypeptide de la protéine de fusion.

Dans une 1^{ère} forme de mise en oeuvre, le polypeptide facilement détectable peut être une enzyme capable de générer un signal détectable et éventuellement quantifiable dans des conditions particulières comme lors de la mise en présence d'un substrat adapté. A titre d'exemples illustratifs et non limitatifs, on peut citer une phosphatase alcaline, une peroxydase, une acétylcholine estérase (AChE), une glucose amylase et une lysozyme.

Dans une 2^{ème} forme de mise en oeuvre, le polypeptide facilement détectable peut être un polypeptide bioluminescent ou fluorescent tel que l'aequorine ; l'obéline ; la luciférase et les protéines fluorescentes telles que la protéine fluorescente verte (GFP), la protéine e-GFP (pour « enhanced GFP »), la protéine fluorescente cyan (eCFP pour « enhanced Cyan Fluorescent Protein »), la protéine fluorescente jaune (eYFP pour « enhanced Yellow Fluorescent Protein »), la protéine fluorescente bleue (eBFP pour « enhanced Blue Fluorescent Protein »), les protéines fluorescentes rouges DsRed et Keima et leurs variants tels que la protéine GFP photoactivable à 405 nm (PA-GFP), la protéine GFP sensible au pH (PHluorin), la protéine Cerulean, la protéine azurite, la protéine Venus, la protéine mCherry et la protéine Citrine.

Par « polypeptide impliqué dans la régulation de l'expression des gènes », on entend, dans le cadre de la présente invention, un polypeptide qui, lorsque présent, augmente ou diminue l'expression d'au moins un gène, comparé à l'expression de ce même gène, dans les mêmes conditions opératoires mais en l'absence de ce polypeptide.

L'homme du métier connaît différents types de polypeptides qui peuvent influencer l'expression d'un (ou plusieurs) gène(s). Avantageusement, un tel polypeptide peut être choisi parmi un facteur de transcription positif ou négatif, un facteur de transcription général, un régulateur de l'expression d'un promoteur, un facteur de remodelage de la chromatine et un facteur modifiant la localisation d'un gène adjacent, un dérivé ou un fragment de ceux-ci.

Ainsi, un polypeptide impliqué dans la régulation de l'expression des gènes peut agir, de façon positive ou négative, directement ou indirectement, soit sur le recrutement du complexe de transcription, soit sur l'état de la chromatine, soit sur la localisation du gène dont l'expression doit être régulée. Un polypeptide agissant sur l'état de la chromatine peut agir sur la décompaction de l'ADN, sur le désenroulement de l'ADN, sur la méthylation des cytosines ou sur l'acétylation des histones. Un polypeptide agissant sur la localisation du gène dont l'expression doit être régulée peut être un facteur permettant d'adresser ou de recruter, à un organite particulier ou une zone cellulaire particulière, le gène adjacent. A titre d'exemples non limitatifs, l'organite particulier ou la zone particulière peuvent être une membrane cellulaire ou la périphérie du noyau. Cet adressage peut impliquer une protéine spécifique d'un organite particulier ou d'une zone cellulaire particulière ou un anticorps monoclonal ou polyclonal dirigé contre une telle protéine, un dérivé ou un fragment de ceux-ci.

Par « dérivé » d'un facteur ou d'un régulateur tel que défini ci-dessus, on entend un polypeptide comprenant au moins 40% de similarité et/ou au moins 30% d'identité avec un facteur ou un régulateur tel que défini ci-dessus connu et présentant une capacité à influer, négativement ou positivement, sur l'expression des gènes. Par « fragment » d'un facteur ou d'un régulateur tel que défini ci-dessus, on entend toute partie ou toute portion d'un facteur ou d'un régulateur tel que défini ci-dessus, ayant conservé sa capacité à influer sur l'expression des gènes. De plus, les formes de mises en oeuvre préférées décrites pour les dérivés et les fragments d'une protéine liant l'ADN et appartenant au système de partition de l'ADN bactérien s'appliquent *mutatis mutandis* aux dérivés et fragments des facteurs ou régulateurs tels que définis ci-dessus.

Plus particulièrement, un polypeptide impliqué dans la régulation de l'expression des gènes est choisi dans la liste d'exemples non exhaustifs ci-dessous :
- les facteurs de transcription TFIIA, TFIIB, TFIID, TRIIE, TFIIF, TFIIH, NF-KappaB, SP1, un facteur de choc thermique,
- une lamine, une protéine membranaire, un anticorps ou un fragment d'anticorps dirigé contre une protéine membranaire ;
- un récepteur nucléaire, tel qu'un récepteur aux oestrogènes, un récepteur à l'acide rétinoïque, un récepteur aux glucocorticoides, un récepteur aux androgènes, un récepteur des 3-kétostéroïdes ou un récepteur des hormones thyroïdiennes ;
- une acétylase, une désacétylase, une méthyl-transférase, une poly ADP-ribosyltransférase, une ubiquitine ligase, une phophatase, une kinase ou une sumoylase.

Dans le vecteur d'expression eucaryote selon la présente invention, la séquence nucléotidique code une protéine fusion comprenant un 1^{er} polypeptide et un 2^{nd} polypeptide tels que précédemment définis, ladite séquence nucléotidique pouvant coder en outre un signal de localisation intracellulaire.

Toutefois, comme les protéines de fusion mises en oeuvre dans le cadre de la présente invention peuvent présenter des dimensions faibles notamment grâce à la taille du 1^{er} polypeptide qu'elles comprennent, la présence d'un signal de localisation intracellulaire n'est pas obligatoire, les protéines de fusion pouvant diffuser librement dans le volume cellulaire et nucléaire.

Lorsque la protéine de fusion comprend un signal de localisation intracellulaire lié de façon opérationnelle aux 1^{er} et 2^{nd} polypeptides tels que précédemment définis, ce dernier peut être un signal de localisation nucléaire. Un tel signal attaché à l'extrémité d'un des deux polypeptides de la protéine de fusion, sans affecter la fonction de l'un ou de l'autre de ces polypeptides favorise le transport de la protéine de fusion dans le noyau cellulaire. L'homme du métier connaît différents signaux de localisation intracellulaire et notamment différents signaux de localisation nucléaire utilisables dans le cadre de la présente invention.

La présente invention concerne également certaines protéines de fusion codées par la séquence nucléotidique du vecteur d'expression eucaryote tel que précédemment défini.

Ainsi, dans une 1^{ère} forme de mise en oeuvre, la protéine de fusion selon l'invention comprend :
- un 1^{er} polypeptide correspondant à une protéine liant l'ADN, sans qu'un quelconque autre facteur ne soit nécessaire à cette liaison, et appartenant au système de partition de l'ADN bactérien, un dérivé ou un fragment de celle-ci notamment tel que précédemment défini et
- un 2^{nd} polypeptide impliqué dans la régulation de l'expression des gènes notamment tel que précédemment défini.

Dans une 2^{nde} forme de mise en oeuvre, la protéine de fusion selon l'invention comprend :
- un 1^{er} polypeptide correspondant à une protéine ParB issue de *Burkholderia cenocepacia,* un dérivé ou un fragment de celle-ci notamment tel que précédemment défini et
- un 2^{nd} polypeptide facilement détectable notamment tel que précédemment défini.

Quelle que soit la forme de mise en oeuvre, la protéine de fusion selon la présente invention peut éventuellement comprendre un signal de localisation intracellulaire, notamment tel que précédemment défini.

Le présent fascicule décrit également un polynucléotide codant une protéine de fusion telle que précédemment définie.

Ainsi, le présent fascicule décrit également un polynucléotide isolé choisi parmi les différents polynucléotides ci-après :
i) un polynucléotide codant une protéine de fusion selon l'invention telle que précédemment définie ;
ii) un polynucléotide complémentaire du polynucléotide tel que défini au point (i) ;
iii) un polynucléotide d'au moins 14 nucléotides et notamment d'au moins 18 nucléotides, capable de s'hybrider dans des conditions de forte stringence aux polynucléotides tels que définis aux points (i) et (ii).

Par « polynucléotide », on entend, dans le cadre de la présente invention un acide nucléique, une séquence nucléique, une séquence d'acide nucléique, un oligonucléotide, une séquence polynucléotidique, une séquence nucléotidique, un ADN simple-brin, un ADN double-brin ou un ARN. Un polynucléotide selon la présente invention comprend des nucléotides naturels et éventuellement des nucléotides non naturels.

Le polynucléotide décrit dans ce fascicule ne correspond pas à une séquence nucléotidique dans son état naturel i.e. dans son environnement chromosomique naturel. Le polynucléotide ne correspond pas non plus à un polynucléotide naturel codant un polypeptide tel que les 1^{er} et 2^{nd} polypeptides de la présente invention. Au contraire, le polynucléotide a été isolé et éventuellement purifié, son environnement a, par conséquent, été modifié. Le polynucléotide peut également être obtenu par recombinaison génétique ou par synthèse chimique.

Les conditions de forte stringence correspondent à des conditions de température et de force ionique qui permettent de maintenir une hybridation entre deux séquences nucléotidiques complémentaires. L'homme du métier saura déterminer les conditions de forte stringence les mieux adaptées notamment en fonction de la taille des séquences nucléotidiques et ce, en se référant à l'enseignement de Sambrook et al. (Molecular cloning, 1989, Noland C. ed., New York : Cold Spring Harbor Laboratory Press). A titre d'exemple illustratif et non limitatif, on entend des conditions de forte stringence de l'étape d'hybridation aux fins de définir les polynucléotides du point (iii) décrit ci-dessus, sont avantageusement les suivantes : une hybridation, notamment de type ADN-ADN ou ADN-ARN, réalisée en deux étapes : (1) préhybridation à 42°C pendant 3 h en tampon phosphate (20 mM, pH 7,5) contenant une solution contenant 0,75 M NaCl, 0,075 M citrate de sodium, 50% de formamide, 7% de sodium dodécyl sulfate (SDS), 10x Denhardt's, 5% de dextran sulfate et 1% d'ADN de sperme de saumon ; (2) hybridation proprement dite pendant 20 h à une température dépendant de la taille de la sonde (par exemple, 42°C pour une sonde de taille > 100 nt) suivie de 2 lavages de 20 min à 20°C dans une solution contenant 0,3 M NaCl, 0,03 M citrate de sodium et 2% SDS ; 1 lavage de 20 min à 20°C dans une solution contenant 0,015 M NaCl, 1,5 mM citrate de sodium et 0,1% SDS. Le dernier lavage est pratiqué dans une solution contenant 0,015 M NaCl, 1,5 mM citrate de sodium et 0,1% SDS pendant 30 min à 60°C pour une sonde de taille > 100 nt.

Le polynucléotide du point (iii) i.e. capable de s'hybrider dans des conditions de forte stringence aux polynucléotides tels que définis aux points (i) et (ii) comprend au moins 20, au moins 50, au moins 100, au moins 150, au moins 200, au moins 250 ou au moins 300 nucléotides.

Le polynucléotide décrit dans ce fascicule comprend au moins une séquence nucléotidique présentant au moins 30%, au moins 40%, au moins 45%, au moins 50%, au moins 55%, au moins 60%, au moins 65%, au moins 70%, au moins 75%, au moins 80%, au moins 85%, au moins 90%, au moins 95% et/ou au moins 99% d'identité avec une des séquences nucléotidiques codant une protéine liant l'ADN et appartenant au système de partition de l'ADN bactérien telle que précédemment définie, notamment une protéine liant l'ADN et appartenant au système de partition de l'ADN bactérien est une protéine ParB issue de *Burkholderia cenocepacia* et, en particulier, la protéine ParB portée par le chromosome 1 de *Bcc* désignée par « ParB-c1 », la protéine ParB portée par le chromosome 2 de *Bcc* désignée par « ParB-c2 », la protéine ParB portée par le chromosome 3 de *Bcc* désignée par « ParB-c3 » et la protéine ParB portée par le plasmide de *Bcc* désignée par « ParB-pl ».

Ainsi, le polynucléotide décrit dans ce fascicule comprend au moins une séquence nucléotidique présentant au moins 30%, au moins 40%, au moins 45%, au moins 50%, au moins 55%, au moins 60%, au moins 65%, au moins 70%, au moins 75%, au moins 80%, au moins 85%, au moins 90%, au moins 95% et/ou au moins 99% d'identité avec la séquence SEQ ID NO: 1 dans le listage de séquences en annexe (i.e. séquence codant la protéine ParB portée par le chromosome 2 de *Bcc* désignée par « ParB-c2 ») ou avec la séquence SEQ ID NO: 3 dans le listage de séquences en annexe (i.e. séquence codant la protéine ParB portée par le chromosome 3 de *Bcc* désignée par « ParB-c2 »).

Il est clair que le polynucléotide couvre également les séquences codant les fragments des protéines liant l'ADN et appartenant au système de partition de l'ADN bactérien précédemment envisagés, les séquences présentant au moins 30%, au moins 40%, au moins 45%, au moins 50%, au moins 55%, au moins 60%, au moins 65%, au moins 70%, au moins 75%, au moins 80%, au moins 85%, au moins 90%, au moins 95% et/ou au moins 99% d'identité avec les séquences codant ces fragments et les séquences complémentaires de telles séquences. A partir des séquences de fragments envisagés et de la séquence nucléotidique codant les protéines liant l'ADN et appartenant au système de partition de l'ADN bactérien, il est facile pour l'homme du métier d'identifier la séquence nucléotidique codant un fragment particulier.

Il est également clair que le polynucléotide couvre également les séquences codant les dérivés des protéines liant l'ADN et appartenant au système de partition de l'ADN bactérien précédemment envisagés, les séquences présentant au moins 30%, au moins 40%, au moins 45%, au moins 50%, au moins 55%, au moins 60%, au moins 65%, au moins 70%, au moins 75%, au moins 80%, au moins 85%, au moins 90%, au moins 95% et/ou au moins 99% d'identité avec les séquences codant ces dérivés et les séquences complémentaires de telles séquences. A partir des séquences de dérivés envisagés et de la séquence nucléotidique codant les protéines liant l'ADN et appartenant au système de partition de l'ADN bactérien, il est facile pour l'homme du métier d'identifier la séquence nucléotidique codant un dérivé particulier.

De plus, le polynucléotide décrit dans ce fascicule comprend au moins une autre séquence nucléotidique codant soit un polypeptide facilement détectable, soit un polypeptide impliqué dans la régulation de l'expression des gènes, tels que précédemment envisagés, les séquences présentant au moins 30%, au moins 40%, au moins 45%, au moins 50%, au moins 55%, au moins 60%, au moins 65%, au moins 70%, au moins 75%, au moins 80%, au moins 85%, au moins 90%, au moins 95% et/ou au moins 99% d'identité avec les séquences codant ces polypeptides et les séquences complémentaires de telles séquences.

Lorsque des acides (ribo)nucléiques sont comparés, leur similarité peut également être déterminée. Dans ce cas, la similarité s'apprécie au niveau des codons. Deux codons sont similaires, s'il s'agit de deux codons différents codant le même acide amine ou de deux codons différents codant deux acides aminés similaires.

Le polynucléotide comprend au moins une séquence nucléotidique présentant au moins 40%, au moins 45%, au moins 50%, au moins 55%, au moins 60%, au moins 65%, au moins 70%, au moins 75%, au moins 80%, au moins 85%, au moins 90%, au moins 95% et/ou au moins 99% de similarité avec une des séquences nucléotidiques codant une protéine liant l'ADN et appartenant au système de partition de l'ADN bactérien telle que précédemment définie, notamment une protéine liant l'ADN et appartenant au système de partition de l'ADN bactérien est une protéine ParB issue de *Burkholderia cenocepacia* et, en particulier, la protéine ParB portée par le chromosome 1 de *Bcc* désignée par « ParB-c1 », la protéine ParB portée par le chromosome 2 de *Bcc* désignée par « ParB-c2 », la protéine ParB portée par le chromosome 3 de *Bcc* désignée par « ParB-c3 » et la protéine ParB portée par le plasmide de *Bcc* désignée par « ParB-pl ».

Ainsi, le polynucléotide comprend au moins une séquence nucléotidique présentant au moins 40%, au moins 45%, au moins 50%, au moins 55%, au moins 60%, au moins 65%, au moins 70%, au moins 75%, au moins 80%, au moins 85%, au moins 90%, au moins 95% et/ou au moins 99% de similarité avec la séquence SEQ ID NO: 1 dans le listage de séquences en annexe (i.e. séquence codant la protéine ParB portée par le chromosome 2 de *Bcc* désignée par « ParB-c2 ») ou avec la séquence SEQ ID NO: 3 dans le listage de séquences en annexe (i.e. séquence codant la protéine ParB portée par le chromosome 3 de *Bcc* désignée par « ParB-c2 »).

Il est clair que le polynucléotide couvre également les séquences codant les fragments des protéines liant l'ADN et appartenant au système de partition de l'ADN bactérien précédemment envisagés, les séquences présentant au moins 40%, au moins 45%, au moins 50%, au moins 55%, au moins 60%, au moins 65%, au moins 70%, au moins 75%, au moins 80%, au moins 85%, au moins 90%, au moins 95% et/ou au moins 99% de similarité avec les séquences codant ces fragments et les séquences complémentaires de telles séquences. A partir des séquences de fragments envisagés et de la séquence nucléotidique codant les protéines liant l'ADN et appartenant au système de partition de l'ADN bactérien, il est facile pour l'homme du métier d'identifier la séquence nucléotidique codant un fragment particulier.

Il est également clair que le polynucléotide couvre également les séquences codant les dérivés des protéines liant l'ADN et appartenant au système de partition de l'ADN bactérien précédemment envisagés, les séquences présentant au moins 40%, au moins 45%, au moins 50%, au moins 55%, au moins 60%, au moins 65%, au moins 70%, au moins 75%, au moins 80%, au moins 85%, au moins 90%, au moins 95% et/ou au moins 99% de similarité avec les séquences codant ces dérivés et les séquences complémentaires de telles séquences. A partir des séquences de dérivés envisagés et de la séquence nucléotidique codant les protéines liant l'ADN et appartenant au système de partition de l'ADN bactérien, il est facile pour l'homme du métier d'identifier la séquence nucléotidique codant un dérivé particulier.

De plus, le polynucléotide décrit dans ce fascicule comprend au moins une autre séquence nucléotidique codant soit un polypeptide facilement détectable, soit un polypeptide impliqué dans la régulation de l'expression des gènes, tels que précédemment envisagés, les séquences présentant au moins 30%, au moins 40%, au moins 45%, au moins 50%, au moins 55%, au moins 60%, au moins 65%, au moins 70%, au moins 75%, au moins 80%, au moins 85%, au moins 90%, au moins 95% et/ou au moins 99% d'identité avec les séquences codant ces polypeptides et les séquences complémentaires de telles séquences.

Enfin, le polynucléotide peut comprendre au moins une autre séquence nucléotidique codant une séquence de localisation intracellulaire notamment telle que précédemment définie.

Le présent fascicule décrit également un vecteur eucaryote comprenant une séquence nucléotidique présentant au moins un site de reconnaissance par une protéine liant l'ADN et appartenant au système de partition de l'ADN bactérien. Le vecteur eucaryote comprend en outre un marqueur de sélection de type eucaryote tel que précédemment défini, présentant au moins un site de reconnaissance par une protéine liant l'ADN et appartenant au système de partition de l'ADN bactérien. Le vecteur eucaryote selon l'invention se présente typiquement sous forme d'un plasmide.

Avantageusement, le vecteur eucaryote est un vecteur intégratif eucaryote. Par « vecteur intégratif eucaryote », on entend un vecteur dont toute ou partie de la séquence nucléotidique est susceptible d'être intégrée dans le génome de l'organisme dans lequel ledit vecteur est inséré. De plus, l'intégration dans le génome de l'organisme hôte peut se faire en un site génomique particulier prédéterminé ou de façon aléatoire. L'intégration ciblée met en oeuvre une recombinaison homologue. Dans ce cas, le vecteur intégratif eucaryote ou un fragment portant ladite séquence produite par PCR, présente des séquences appropriées, de part et d'autre de la séquence nucléotidique présentant au moins un site de reconnaissance par une protéine liant l'ADN et appartenant au système de partition de l'ADN bactérien, et ce, pour permettre cette recombinaison homologue.

Tout site de reconnaissance auquel se lie une protéine appartenant au système de partition de l'ADN plasmidique ou chromosomique d'un procaryote est utilisable dans le cadre de la présente invention. L'article de Dubarry et al, 2006 décrit différents sites de reconnaissance reconnus par différentes protéines liant l'ADN et appartenant au système de partition de l'ADN bactérien. Tous ces sites de reconnaissance sont dans la portée de la présente invention. Le site de reconnaissance mis en oeuvre dans le cadre de la présente invention est, de préférence, double-brin.

Avantageusement, le site de reconnaissance par une protéine liant l'ADN et appartenant au système de partition de l'ADN bactérien, mis en oeuvre dans le cadre de la présente invention est de séquence nucléotidique (I) ou de séquence complémentaire à la séquence nucléotidique (I) :
N₁N₂TN₃N₄N₅N₆CGN₇N₈N₉N₁₀AN₁₁N₁₂ (I) (SEQ ID NO: 5 dans le listage de séquences en annexe) dans laquelle les nucléotides N₁ et N₁₂, identiques ou différents, sont choisis parmi A, G, C ou T et les couples de nucléotides (N₆,N₇), (N₅,N₈), (N₄,N₉), (N₃,N₁₀) et (N₂,N₁₁) indépendamment les uns des autres sont choisis dans le groupe constitué par (A,T), (T,A), (C,G) et (G,C), les nucléotides N₁ et N₁₂ pouvant éventuellement être absents.

Avantageusement, dans la séquence (I) ci-dessus, N₁ est absent ou représente G, C ou T ; le couple (N₆,N₇) représente (A,T), (T,A) ou (G,C) ; le couple (N₅,N₈) représente (C,G) ou (T,A) ; le couple (N₄,N₉) représente (A,T), (T,A), (C,G) ou (G,C) ; le couple (N₃,N₁₀) représente (G,C) ou (T,A) ; le couple (N₂,N₁₁) représente (T,A) ou (G,C) et N₁₂ est absent ou représente A ou C.

En effet, dans le site de reconnaissance, la nature symétrique (N₂ à N₆ avec N₇ à N₁₁) est très importante pour la reconnaissance. Il se peut donc qu'une séquence de même organisation symétrique, sans forcément d'identité nucléotidique, puisse avoir les mêmes caractéristiques.

En particulier, le site de reconnaissance par une protéine liant l'ADN et appartenant au système de partition de l'ADN bactérien, mis en oeuvre dans le cadre de la présente invention est de séquence nucléotidique (II) ou de séquence complémentaire à la séquence nucléotidique (II) :
N₁₃TTN₁₄N₁₅N₁₆N₁₇CGN₁₈N₁₉N₂₀N₂₁AAC (II) (SEQ ID NO: 6 dans le listage de séquences en annexe) dans laquelle :
- N₁₃ représente G, C ou T ;
- le couple (N₁₄,N₂₁) représente (T,A) ou (G, C) ;
- le couple (N₁₅,N₂₀) représente (A,T) ou (T,A) ;
- le couple (N₁₆,N₁₉) représente (T,A) ou (C,G) ;
- le couple (N₁₇,N₁₈) représente (G,C) ou (A,T) .

Avantageusement, le site de reconnaissance par une protéine liant l'ADN et appartenant au système de partition de l'ADN bactérien présente une séquence nucléotidique choisie parmi les séquences nucléotidiques suivantes :
- GTTTATGCGCATAAAC (Sc2 ; SEQ ID NO: 7 dans le listage de séquences en annexe) ;
- CTTTATGCGCATAAAC (Sc2 ; SEQ ID NO: 8 dans le listage de séquences en annexe) ;
- GTTGTCACGTGACAAC (Sc3 ; SEQ ID NO: 9 dans le listage de séquences en annexe) ;
- TTTGTCACGTGACAAC (Sc3 ; SEQ ID NO: 10 dans le listage de séquences en annexe) ;
- CTTGTCACGTGACAAC (Sc3 ; SEQ ID NO: 11 dans le listage de séquences en annexe) ;
et une séquence complémentaire à l'une quelconque de ces séquences.

La séquence nucléotidique du vecteur eucaryote éventuellement intégratif décrit dans ce fascicule comprend au moins 2, au moins 3 ou au moins 4 sites de reconnaissance par une protéine liant l'ADN et appartenant au système de partition de l'ADN bactérien tels que précédemment définis. Lorsque le polynucléotide selon la présente invention comprend au moins 2 sites de reconnaissance, deux sites de reconnaissance consécutifs peuvent être identiques ou différents l'un de l'autre. Lorsque le polynucléotide selon la présente invention comprend au moins 2 sites de reconnaissance, deux sites de reconnaissance consécutifs peuvent être séparés par au moins 1 nucléotide, notamment au moins 10 nucléotides, en particulier, au moins 50 nucléotides et, plus particulièrement, au moins 100 nucléotides.

La séquence nucléotidique du vecteur eucaryote éventuellement intégratif peut être naturelle, synthétique ou avoir été obtenue par mutation d'une séquence naturelle.

La séquence nucléotidique du vecteur eucaryote éventuellement intégratif comprend avantageusement une séquence centromérique impliquée dans le système de partition bactérien. En particulier, cette séquence centromérique peut être une séquence provenant d'une quelconque bactérie telle que *Bacillus subtilis, Caulobacter crescentus* etc...

Dans une forme de mise en oeuvre particulière, la séquence nucléotidique du vecteur eucaryote éventuellement intégratif comprend avantageusement une séquence centromérique impliquée dans le système de partition de la bactérie *Burkholderia cenocepacia* (*Bcc*), séquence connue sous le nom de ParS. Plus particulièrement encore, cette séquence nucléotidique correspond à une séquence choisie parmi :
- la séquence ParS portée par le chromosome 1 de *Bcc* désignée par « ParS-c1 » qui peut être obtenue à partir de la séquence nucléotidique du chromosome 1 de la souche J2315 de *Bcc* accessible dans Genbank sous le N° AM 747720, les deux sites de reconnaissance que contient cette séquence se trouvant respectivement aux nucléotides 26477-26492 et 28403-28418 ;
- la séquence ParS portée par le chromosome 2 de *Bcc* désignée par « ParS-c2 » qui peut être obtenue à partir de la séquence nucléotidique du chromosome 2 de la souche J2315 de *Bcc* accessible dans Genbank sous le N° AM 747721 et qui correspondant à la protéine SEQ ID NO: 12 dans le listage de séquences en annexe ;
- la séquence ParS portée par le chromosome 3 de *Bcc* désignée par « ParS-c3 » qui peut être obtenue à partir de la séquence nucléotidique du chromosome 3 de la souche J2315 de *Bcc* accessible dans Genbank sous le N° AM 747722 et qui correspondant à la protéine SEQ ID NO: 13 dans le listage de séquences en annexe ;
- la séquence ParS portée par le plasmide de *Bcc* désignée par « ParS-pl » qui peut être obtenue à partir de la séquence nucléotidique du plasmide pBCJ2315 de la souche J2315 de *Bcc* accessible dans Genbank sous le N° AM 747723, les trois sites de reconnaissance que contient cette séquence se trouvant respectivement aux nucléotides 92285-92300, 92385-92400 et 92438-92453 ;
ou une séquence complémentaire à l'une quelconque de ces séquences.

Le fascicule décrit également un organisme hôte eucaryote, transformé par ou comprenant un vecteur d'expression eucaryote tel que précédemment défini ou un vecteur eucaryote éventuellement intégratif tel que précédemment défini.

Par « organisme hôte eucaryote », on entend tout organisme eucaryote, uni- ou pluricellulaire, inférieur ou supérieur, dans lequel un vecteur d'expression eucaryote tel que précédemment défini ou un vecteur intégratif eucaryote tel que précédemment défini est introduit.

Ainsi, un organisme hôte eucaryote peut être un micro-organisme tel qu'une levure ou un champignon.

En variante, un organisme hôte eucaryote peut être une cellule animale telle qu'une cellule d'insecte ou une cellule de mammifère et notamment une cellule humaine, une cellule de hamster, une cellule de singe, une cellule de lapin, une cellule de souris, une cellule de rat, etc... ; une cellule végétale ; une plante ; ou un animal à l'exception d'un humain. De fait, l'organisme hôte peut être une plante transgénique ou un animal transgénique à l'exception d'un humain.

L'organisme hôte de type cellule peut être fourni sous forme d'une culture en suspension, d'une culture sur boîtes de culture, d'une culture tissulaire ou d'une culture d'organe.

L'homme du métier connaît différentes méthodes de transformation ou de transfection pour introduire de façon efficace un vecteur d'expression eucaryote tel que précédemment défini ou un vecteur intégratif eucaryote tel que précédemment défini dans un organisme hôte. L'introduction du vecteur d'expression selon l'invention aboutit à la production d'une protéine de fusion telle que précédemment définie.

A titre d'exemple et de façon non exhaustive, cette méthode peut être une électroporation ; une lipofection ; une micro-injection ; un bombardement avec des particules (ou biolistique) ; une transformation biologique d'un végétal en utilisant *Agrobacterium tumefasciens* ; une transformation par une perméabilisation chimique via une augmentation de la température, un choc thermique, un traitement par des détergents ou un traitement par du polyéthylèneglycol ; une transformation par la méthode DEAE-dextran ou une introduction via un virus, un virion ou une particule virale.

Avantageusement, l'organisme hôte est un organisme hôte transformé par ou comprenant un vecteur d'expression eucaryote tel que précédemment défini et un vecteur eucaryote éventuellement intégratif tel que précédemment défini.

En particulier, l'organisme hôte est un organisme hôte ayant intégré, dans son génome ou dans de l'ADN exogène qu'il contient comme un plasmide ou un transposon, la séquence nucléotidique présentant au moins un site de reconnaissance par une protéine liant l'ADN et appartenant au système de partition de l'ADN bactérien, telle que précédemment définie (i.e. la séquence nucléotidique présente sur le vecteur intégratif eucaryote selon l'invention) et exprimant la protéine de fusion telle que précédemment définie (i.e. la protéine de fusion codée par la séquence nucléotidique présente sur le vecteur d'expression eucaryote selon l'invention).

Plus particulièrement, l'organisme hôte exprime de façon transitoire la protéine de fusion telle que précédemment définie. En variante, l'organisme hôte exprime de façon constitutive la protéine de fusion telle que précédemment définie.

Comme précédemment explicité, la séquence nucléotidique présentant au moins un site de reconnaissance par une protéine liant l'ADN et appartenant au système de partition de l'ADN bactérien, telle que précédemment définie peut être introduite dans l'organisme hôte en un site particulier prédéterminé en utilisant la recombinaison homologue. Avantageusement, dans ce cas, l'organisme hôte est une levure. Dans ce cas également, le gène adjacent au site d'insertion de la séquence nucléotidique présentant au moins un site de reconnaissance par une protéine liant l'ADN et appartenant au système de partition de l'ADN bactérien est connu.

En variante, l'insertion dans le génome de l'organisme hôte peut se faire de manière aléatoire. Dans ce cas, l'homme du métier peut déterminer, par des techniques classiques de biologie moléculaire, la position de cette insertion. Connaissant cette position, l'homme du métier peut sélectionner un organisme hôte dans lequel l'insertion a eu lieu au niveau d'un site d'intérêt. L'organisme hôte ainsi sélectionné peut générer une lignée cellulaire stable telle qu'une lignée cellulaire stable de mammifères.

Que l'insertion se fasse de façon dirigée ou aléatoire, il est ainsi possible d'obtenir un organisme hôte dans lequel l'insertion a eu lieu au niveau d'un ou plusieurs sites d'intérêt tel qu'un site proche d'un oncogène, proche d'un suppresseur de tumeur, proche d'un gène impliqué dans une maladie génétique etc....

Si, dans ce cas, la protéine de fusion exprimée par l'organisme hôte comprend un polypeptide impliqué dans la régulation de l'expression des gènes notamment tel que précédemment défini, il est possible de contrôler, de façon positive ou négative, l'expression du gène adjacent au site d'insertion de la séquence nucléotidique présentant au moins un site de reconnaissance par une protéine liant l'ADN et appartenant au système de partition de l'ADN bactérien.

De même, il est possible de détecter une molécule susceptible de contrôler cette expression. Par « molécule susceptible de contrôler cette expression », une molécule naturelle ou synthétique, notamment une molécule biologique ou biologiquement active, qui peut être apte à influencer l'expression d'un gène. Avantageusement, cette molécule est choisie parmi les épitopes ; les antigènes ; les peptides ; les oligopeptides ; les protéines telles qu'une enzyme ; les anticorps et fragments d'anticorps ; les récepteurs cellulaires ou membranaires ; des polysaccharides ; et les molécules nucléiques telles qu'un ADN simple ou double brin, un ARN, un ARNi, un miRNA, un aptamère, un PNA (pour « peptide nucleic acid ») ou un LNA (pour « locked nucleic acid »). Une telle molécule peut avoir des applications dans le domaine thérapeutique.

En effet, la présente invention propose un procédé pour détecter une molécule susceptible de contrôler, de façon positive ou négative, l'expression d'un gène d'intérêt. Ce procédé comprend :
- la mise en contact de ladite molécule avec un organisme hôte selon la présente invention dans lequel une protéine de fusion comprenant un polypeptide impliqué dans la régulation de l'expression des gènes selon l'invention est exprimée et dans lequel la séquence nucléotidique présentant au moins un site de reconnaissance par une protéine liant l'ADN et appartenant au système de partition de l'ADN bactérien a été insérée proche du gène d'intérêt ;
- la détection de l'expression de ce gène d'intérêt et la comparaison avec l'expression obtenue en l'absence de ladite molécule.

Si l'expression est identique que la molécule testée soit présente ou non, cette dernière n'a pas d'effet sur cette expression. Au contraire, si une différence existe entre les deux expressions, la molécule testée influence, de façon directe ou indirecte et de façon positive ou négative, cette expression.

En variante, si la protéine de fusion exprimée par l'organisme hôte comprend un polypeptide facilement détectable, il est possible de détecter, *in vivo,* la position d'un site d'intérêt de l'ADN génomique ou d'un ADN exogène que contient l'organisme hôte, comme un plasmide ou un transposon, et sa dynamique en conditions normales ou en présence d'une molécule susceptible d'affecter cette position.

Par « molécule susceptible d'affecter cette position », on entend une molécule naturelle ou synthétique, notamment une molécule biologique ou biologiquement active, qui peut être apte à modifier la position d'un gène. Avantageusement, cette molécule est choisie parmi les épitopes ; les antigènes ; les peptides ; les oligopeptides ; les protéines telles qu'une enzyme ; les anticorps et fragments d'anticorps ; les récepteurs cellulaires ou membranaires ; des polysaccharides ; et les molécules nucléiques telles qu'un ADN simple ou double brin, un ARN, un ARNi, un aptamère, un PNA ou un LNA.

En effet, la présente invention propose un procédé pour détecter une molécule susceptible d'affecter la position d'un gène ou d'un site d'intérêt. Ce procédé comprend :
- la mise en contact de ladite molécule avec un organisme hôte selon la présente invention dans lequel une protéine de fusion comprenant un polypeptide facilement détectable selon l'invention est exprimée et dans lequel la séquence nucléotidique présentant au moins un site de reconnaissance par une protéine liant l'ADN et appartenant au système de partition de l'ADN bactérien a été insérée proche du gène ou du site d'intérêt ;
- la détection du polypeptide facilement détectable et donc la position du site de reconnaissance par une protéine liant l'ADN et appartenant au système de partition de l'ADN bactérien et donc encore la position du gène ou du site d'intérêt et la comparaison avec la position du gène ou du site d'intérêt obtenue en l'absence de ladite molécule.

Si la position est identique que la molécule testée soit présente ou non, cette dernière n'a pas d'effet sur cette expression. Au contraire, si une différence existe entre les deux positions, la molécule testée influence, de façon directe ou indirecte, cette position.

Dans une forme de mise en oeuvre particulière, le site d'intérêt correspond à une cassure double-brin de l'ADN. Dans ce cas, le suivi de la position de ce site d'intérêt permet d'étudier la résection de l'ADN lors de la réparation de cette cassure double-brin (disparition du signal détectable émis par le polypeptide facilement détectable) et de détecter des molécules impliquées dans cette dégradation ou influant sur ce processus. En variante encore, le procédé selon l'invention permet de suivre au moins une des deux extrémités de la cassure double-brin et de fait de détecter une molécule impliquée dans le maintien des extrémités de l'ADN flanquant le site de coupure. Les molécules identifiées dans cette forme de mise en oeuvre particulière peuvent présenter un intérêt thérapeutique, notamment dans le domaine de la cancérologie et de la réponse aux traitements anticancéreux, génotoxiques etc.

En variante, la présente invention concerne un procédé pour étudier la dynamique d'un site ou d'un gène d'intérêt de l'ADN génomique ou exogène que contient l'organisme hôte. Ce procédé comprend :
- la fourniture d'un organisme hôte selon la présente invention dans lequel une protéine de fusion comprenant un polypeptide facilement détectable selon l'invention est exprimée et dans lequel la séquence nucléotidique présentant au moins un site de reconnaissance par une protéine liant l'ADN et appartenant au système de partition de l'ADN bactérien a été insérée proche du gène ou du site d'intérêt ;
- la détection, à au moins deux temps différents, désignés T₁ et T₂, avec T₂ > T₁, du polypeptide facilement détectable et donc la position du site de reconnaissance par une protéine liant l'ADN et appartenant au système de partition de l'ADN bactérien et donc encore la position du gène ou du site d'intérêt et la comparaison de la position du gène ou du site d'intérêt obtenue au temps T₁ avec celle obtenue au temps T₂.

Dans cette forme de mise en oeuvre particulière, la détection peut être réalisée en continue ou de façon ponctuelle à différents temps désignés Tₙ avec n représentant un entier compris entre 1 et 10000, chaque temps Tₓ étant séparé du temps Tₓ₊₁ avec x représentant un entier compris entre 1 et 9999, par une durée comprise entre 1 ms et 10 h, cette durée de séparation pouvant être constante ou variable.

Par conséquent, la présente invention concerne l'utilisation non thérapeutique d'un élément de l'invention choisi parmi un vecteur d'expression eucaryote tel que précédemment défini, une protéine de fusion telle que précédemment définie, un vecteur eucaryote éventuellement intégratif tel que précédemment défini et un organisme hôte tel que précédemment défini pour contrôler l'expression d'un gène *in vivo* chez un eucaryote à l'exception d'un humain ou pour détecter la position, la dynamique ou la dégradation (ou le métabolisme) de loci d'ADN ou la recombinaison ou l'échange de séquences d'ADN *in vivo* chez un eucaryote.

La présente invention concerne aussi l'utilisation d'au moins un élément de l'invention choisi parmi un vecteur d'expression eucaryote tel que précédemment défini, une protéine de fusion telle que précédemment définie, une séquence nucléotidique telle que précédemment définie, un vecteur eucaryote éventuellement intégratif tel que précédemment défini et un organisme hôte tel que précédemment défini pour détecter une molécule susceptible de contrôler l'expression d'un gène *in vivo* chez un eucaryote ou susceptible d'affecter la position, la dynamique ou la dégradation (ou le métabolisme) de loci d'ADN ou la recombinaison ou l'échange de séquences d'ADN *in vivo* chez un eucaryote.

Dans ces utilisations du système objet de la présente invention, les loci et séquences d'ADN peuvent être soit endogènes, soit exogènes et notamment sur un plasmide ou un transposon.

D'autres caractéristiques et avantages de la présente invention apparaîtront encore à l'homme du métier à la lecture des exemples ci-dessous donnés à titre illustratif et non limitatif, en référence aux figures annexées.

### BRÈVE DESCRIPTION DES DESSINS

La Figure 1 présente le diagramme circulaire du plasmide pMLBADcat-ParB-c2 :: mCherry (Figure 1A), du plasmide pMLBADcat-ParB-c2 ::eGFP (Figure 1B), du plasmide pDAG512 (Figure 1C) et du plasmide pDAG514 (Figure 1D).
La Figure 2 présente le schéma des vecteurs contenant les fusions ParB pour une expression dans les levures.
La Figure 3 présente le schéma des séquences centromériques ParS-c2 et ParS-c3.
La Figure 4 présente le schéma des vecteurs d'expression des protéines de fusion ParB dans les cellules de mammifères.
La Figure 5 présente le schéma des séquences centromériques ParS-c2 and ParS-c3 pour l'intégration dans les cellules de mammifères.
La Figure 6 présente des photographies de cellules de levure exprimant la construction pCM189 ParB-c2-mCherry avec la construction ParS-c2 insérée dans le génome (ParS-c2) ou non (WT).
La Figure 7 présente des photographies de cellules humaines HeLa exprimant de façon transitoire soit la construction peGFP-c2 ParB-c3-GFP ou peGFP-c2 ParB-c2-mCherry et co-transfectées avec de l'eau (Mock) ou avec un plasmide qui contient les séquences ParS-c2 ou ParS-c3 (ParS). Barres 15 µm.
La Figure 8 présente des photographies de cellules humaines HeLa ayant stablement intégré une construction ParS-c2 unique et exprimant de façon transitoire ParB-mcherry à partir du plasmide pFG9.
La Figure 9 présente des exemples de mouvement du locus *HML* au cours du temps en utilisant le système de localisation ParS-c2/ParB-c2-mCherry.
La Figure 10 présente le suivi de la position de l'étiquette fluorescente à *HML* en fonction du temps. La Figure 10A présente le trajet de *HML* durant l'acquisition. La Figure 10B montre la caractérisation du mouvement de *HML* par analyse de *Mean-Square displacement* (MSD).
La Figure 11 présente la dégradation de la séquence ParS-c2 insérée dans le locus *MAT* lors de l'induction d'une cassure double brin unique par l'endonucléase HO. La séquence ParS-c2 est suivie via ParB-c2-mCherry (ParB-c2-mCherry) et le locus MAT est suivi (Mat-YFP). Barres 2 µm.
La Figure 12 présente la quantification du signal de fluorescence des cellules présentées à la Figure 11.

### EXPOSÉ DÉTAILLÉ DE MODES DE RÉALISATION PARTICULIERS

### I. Techniques mises en oeuvre.

### I.1. Construction des vecteurs d'expression ParB.

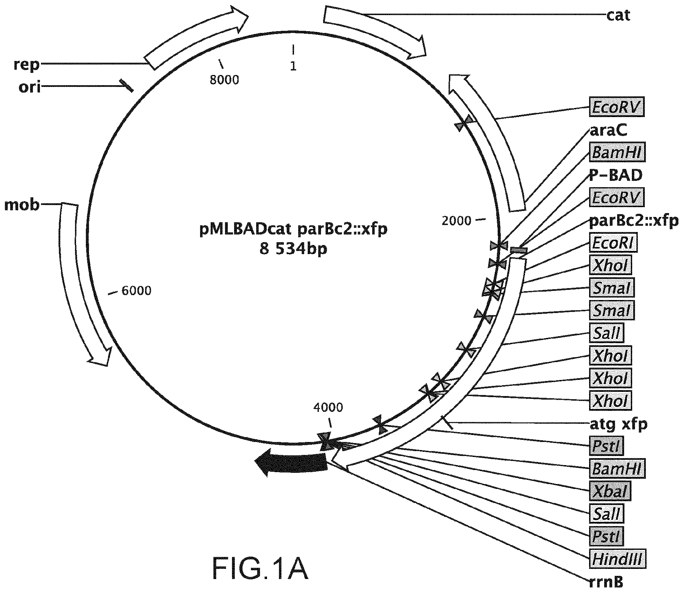

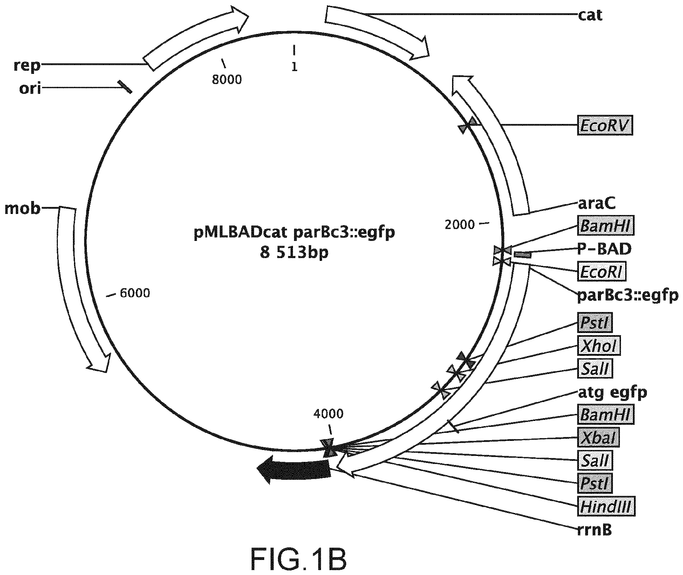

ParB-c2-mCherry et ParB-c3-GFP ont été amplifiés par PCR à partir respectivement des plasmides pMLBADcat-ParB-c2 :: mCherry (Figure 1A) et pMLBADcat-ParB-c2 :: eGFP (Figure 1B) et les amorces E et F suivantes :
E : 5'-ATTAGC*GGATCC*TACCTGACGCTTTTTA-3' (SEQ ID NO: 14 dans le listage de séquences en annexe) et
F : 5'-GAATT*GCGGCCGC*TTACTTGTACAGCTCGTCCA-3' (SEQ ID NO: 15 dans le listage de séquences en annexe).

Les produits de PCR ont été digérés BamH1/Not1 puis insérés dans les vecteurs d'expression de levure pCM189 pour ParB-c2-mCherry et pCM184 pour ParB-c3-GFP digérés par BamH1/Not1 pour créer pFG7 et pFG8.

Pour les cellules de mammifères, le plasmide peGFP-c2 de *Clontech*© (GenBank Accession #: U57606) a été digéré par Nhe1/HindIII pour enlever la séquence codante de la GFP. Un fragment Nhe1/HindIII contenant soit ParB-c2-mCherry ou ParB-c3-GFP des plasmides pMLBADcat-ParB-c2 :: mCherry ou pMLBADcat-ParB-c2 :: eGFP a ensuite été inséré dans les plasmides digérés peGFP-c2 pour créer pFG9 et pFG10.

### I.2. Construction des plasmides contenant les séquences ParS.

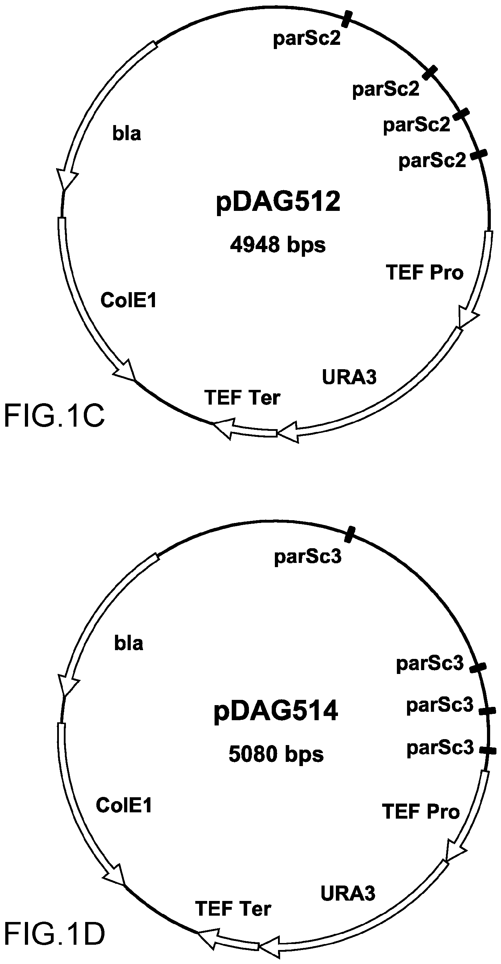

Les plasmides contenant les séquences bactériennes ParS-c2 et ParS-c3 ont été précédemment clonés (Dubarry et al, 2006) . Les plasmides navette de levure pDAG512 (ParS-c2 ; Figure 1C) et pDAG514 (ParS-c3 ; Figure 1D) ont été digérés par BglII/Spe1 afin d'exciser le marqueur *URA3.*

Les vecteurs linéaires ont ensuite été associés avec un fragment BglII/Spe1 contenant soit le marqueur *NAT* soit le marqueur *Hygro* des plasmides pAG25 et pAG32, respectivement, pour créer les plasmides pFG2 et pFG4.

Pour les vecteurs de cellules de mammifères, un fragment BamH1/BglII contenant ParS-c2 ou ParS-c3 a été ligué dans un vecteur pMSCV-Hygro (*Clontech* ©, Cat. No. 634401) digéré par BamH1 pour créer pFG5 et pFG6.

### I.3. Amplification des séquences ParS pour intégration.

Pour l'intégration dans les loci *HML* ou *MAT,* les oligonucléotides G et H ont été utilisés en PCR avec pFG2 comme matrice :
G : 5'-CTTCAAAGAAATATTTAAACTCATTTATGGCTTTTAGAGC ATATTACTCAGTGACACTATAGAACGCGGCCGCCA-3' (SEQ ID NO: 16 dans le listage de séquences en annexe) et
H : 5'-TCAGCGAGCAGAGAAGACAAGACATTTTGTTTTACACCGG AGCCAAACTGTATAGGGAGACCGGCAGATCCGCGG-3' (SEQ ID NO: 17 dans le listage de séquences en annexe).

Un mélange de Taq et Phusion (2/1 U) ADN polymérase a été utilisé pour l'amplification des séquences ParS-*NAT* dans 100 µl de tampon 1x Go Taq, 2% DMSO, 200 µM dNTP, 1 µM de chaque amorce.

La réaction de PCR a été réalisée comme suit : 98°C 2 min, 35 cycles à 98°C 30 sec, 54°C 30 sec, 72°C 2,5 min suivie d'une période de 10 min à 72°C. Les produits d'amplification ont ensuite été purifiés en utilisant un kit de purification de PCR Quiagen. 5 µg a été utilisé pour transformer une souche de levure sauvage de fond génétique W303.

### I.4. Intégration des séquences ParS-NAT.

Les cellules de levure ont été transformées avec le protocole de Gietz et al. (Gietz & Schiestl, 1991). Après transformation, les cellules ont été étalées sur des boîtes YPD pour 18 h afin de permettre l'expression du marqueur *NAT.* Les cellules ont été répliquées sur des boîtes de YPD contenant de la Nourseothricine à une concentration finale de 200 µg/ml.

Après deux étapes de sélection NAT, des clones résistants ont été sélectionnés pour extraction d'ADN génomique et l'intégration des séquences ParS a été vérifiée par PCR. Les clones possédant une intégration correcte ont ensuite été transformés par pFG7 et mis en culture dans du milieu minimum pour réaliser la microscopie sur les cellules vivantes.

Pour l'intégration dans *MAT,* une souche *YIL11,* contenant un opérateur/répresseur-YFP en position 197 kb en amont de *MAT* a été utilisée pour intégrer une séquence ParS-c2 à 100 nt du site de coupure de l'endonucléase HO. Les cellules ont ensuite été transformées par un plasmide portant une version inductible de l'endonucléase HO et par pCM189 ParB-c2-mCherry.

### I.5. Transfection des cellules Hela.

Des cellules Hela au 5^{ième} passage (*ATCC*) ont été ensemencées dans des boîtes 6-puits à une concentration de 400000 cellules/puits dans 2 ml de milieu DMEM, 10% de sérum de veau foetal (SVF), 1X Pyruvate de sodium.

Les cellules ont proliféré pendant 24 h à 37°C, 5% CO₂. A 70% de confluence, la transfection a été effectuée. Brièvement, 100 µl de Opti-MEM contenant 1,5 µg de chaque plasmide et 10 µl de réactif de transfection FuGene HD (Roche) ont été mélangés et la création de liposome a été effectuée 20 min à température ambiante. Le mélange de transfection a ensuite été ajouté sur les cellules au goutte à goutte.

Dans les conditions expérimentales mises en oeuvre, une période d'incubation de 20 h post-transfection est suffisante pour obtenir un bon signal de fluorescence.

### I.6. Transfection des cellules Hela.

Des cellules HeLa au 8^{ième} passage ont été ensemencées dans des boîtes de 100 mm à une concentration de 2,5 10⁶ cellules dans 10 ml de milieu DMEM, 10% de SVF, 1X Pyruvate de sodium. 500 µl de Opti-MEM contenant 15 µg de chaque produit de PCR à intégrer et 33,3 µl de réactif de transfection FuGene HD (Roche) ont été mélangés et la création de liposomes a été effectuée 20 min à température ambiante. Le mélange de transfection a ensuite été ajouté sur les cellules au goutte à goutte.

5h post-transfection, du sulfate de bléomycine a été ajouté pour 20 h à une concentration de 0,1 µM. 20h après l'ajout de bléomycine, les cellules ont été lavées deux fois au PBS puis 10 ml de milieu contenant 200 µg/ml d'hygromycine a été ajouté. Les cellules ont été incubées pendant plusieurs jours en changeant le milieu tous les 3 jours jusqu'à ce que les cellules témoins non-transfectées soient toutes contre sélectionnées.

Les clones résistants ont ensuite été concentrés par passage successif sur boîte de 35 mm, 60 mm, 100 mm et 140 mm puis congelés. Les clones stables ont été ensemencés dans des boîtes 6-puits à une concentration de 400000 cellules/puits dans 2 ml de milieu DMEM, 10% SVF, 1X Pyruvate de sodium. Les cellules ont poussé pendant 24 h à 37°C, 5% CO₂. A 70% confluence, la transfection a été effectuée. Brièvement, 100 µl de Opti-MEM contenant 1,5 µg de chaque plasmide et 10 µl de réactif de transfection FuGene HD (Roche) ont été mélangés et la création de liposomes a été effectuée 20 min à température ambiante. Le mélange de transfection a ensuite été ajouté sur les cellules au goutte à goutte. L'imagerie des foci ParB a été réalisée 24 h post-transfection.

### I.7. Acquisition des images.

Pour les levures, les cellules ont été mises en culture jusqu'à ce qu'elles atteignent le milieu de la phase exponentielle de croissance dans du milieu sélectif YNBD.

Les images ont été acquises en utilisant un microscope inversé à fluorescence Olympus IX-81 équipé avec une caméra CoolSnapHQ (Princeton Instrument) et un objectif 100X PL APO NA 1,4. Le temps d'exposition pour ParB-c2-mCherry est de 1000 ms. L'acquisition sur les cellules a été réalisée par acquisition séquentielle en temps réel (une image par seconde pendant 50 sec).

Les images des cellules humaines ont été acquises sur un microscope inversé à fluorescence Nikon T5100 avec un objectif 40X PL APO NA 0,95 et une caméra Hamamatsu. Les temps d'acquisition sont compris entre 400 ms et 1000 ms.

### I.8. Mesure de la dynamique des particules.

Les piles d'images ont été importées dans le programme Image J (http://rsbweb.nih.gov/ij/) et la position des particules a été déterminée automatiquement en fonction du temps en utilisant le plug-in *Particle detector and tracker* réglé avec les paramètres suivants : Radius = 4, CutOff = 0, percentile 0,1, link = 2, displacement = 8.

Les trajectoires générées ont été inspectées visuellement pour vérifier la précision du suivi des particules. La vitesse et le déplacement quadratique moyen (MSD) *ont* été calculés mathématiquement en utilisant les formules : 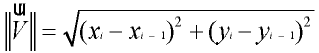 $MSD = {\sum }_{0 - i} {\left[\sqrt{{\left({x}_{i}-{x}_{0}\right)}^{2} + {\left({y}_{i}-{y}_{0}\right)}^{2}}-\sqrt{{\left({x}_{i - 1}-{x}_{0}\right)}^{2} + {\left({y}_{i - 1}-{y}_{0}\right)}^{2}}\right]}^{2}$

### II. Résultats.

### II.1. Constructions générées.

### A. Remarques préalables.

Est utilisée la séquence d'ADN correspondant aux sites endogènes ParS-c2 et ParS-c3 présents respectivement dans les chromosomes 2 et 3 de *Bcc.* Chaque séquence ParS contient quatre sites de liaison de protéines ParB (i.e. sites de reconnaissance).

Les marqueurs de sélection disponibles pour l'intégration de ces séquences sont les gènes de résistance à la nourséothricine et à l'hygromycine pour les cellules de levure et le gène de résistance à l'hygromycine pour les cellules de mammifères.

Les séquences d'intégration ParS sont aisément produites par PCR. Cette amplification permet une intégration ciblée dans les cellules de levure et aléatoire dans les cellules de mammifères.

Est également fourni un acide nucléique qui encode une protéine de fusion. Cette construction contient soit la séquence codante de ParB-c2 ou ParB-c3 fusionnée à un rapporteur fluorescent, soit la GFP ou son variant mCherry.

Ces acides nucléiques sont clonés dans des vecteurs d'expression spécifiques de levure ou de mammifère.

Pour les levures, les vecteurs sont dérivés des plasmides pCM184 et pCM189 qui contiennent des promoteurs Tet OFF permettant la régulation de l'expression de la séquence insérée en aval par ajout de doxycycline (Belli et al, 1998; Gari et al, 1997). En l'absence de doxycycline, l'expression de la construction est maximale.

Pour les cellules de mammifères, le vecteur est dérivé du plasmide peGFP-c2 de *Clontech*© (GenBank Accession #: U57606). Ce plasmide contient un promoteur CMV qui entraîne une forte expression de la protéine de fusion de façon dépendante du temps. Les fusions ParB ne contiennent aucun signal de localisation intracellulaire mais grâce à leur faible dimension (< 70 kDa), elles diffusent librement dans le volume cellulaire et nucléaire.

### B. Pour utilisation chez la levure.

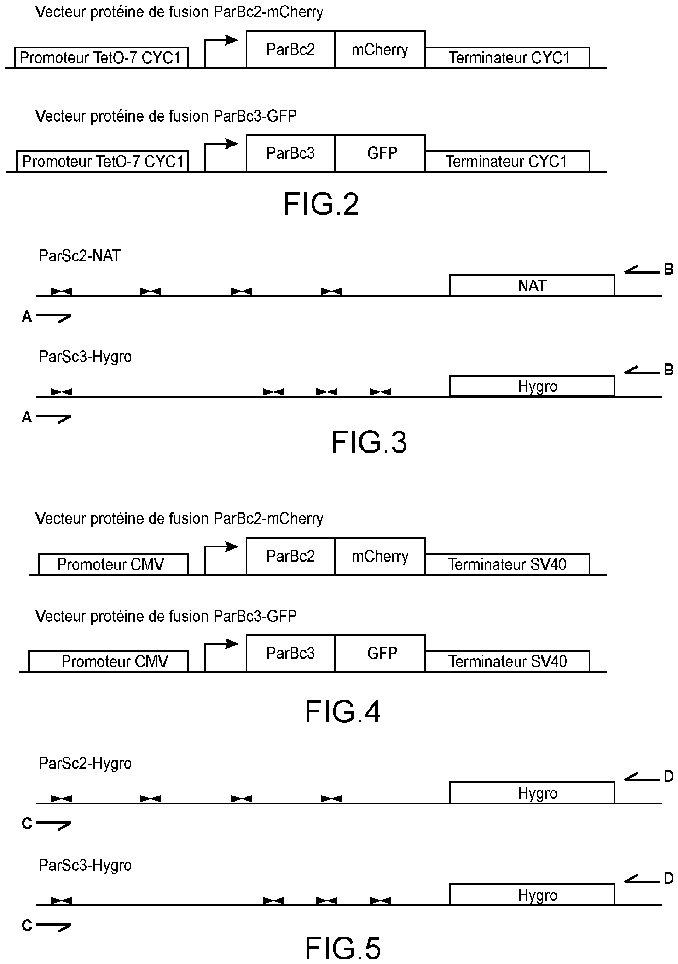

Les deux constructions ParB-c2-mCherry et ParB-c3-GFP utilisées chez la levure sont sous le contrôle du promoteur CYC1-TetO-7 (Figure 2). Ce promoteur contient sept TeT-Opérateurs qui sont liés spécifiquement par un Transactivateur de transcription (tTA) encodé sur le même plasmide. L'ajout de doxycycline induit la répression de l'expression de la construction de manière dose dépendante.

Les séquences ParS (Figure 3) sont insérées dans l'ADN génomique par amplification par PCR en utilisant les couples d'amorces A et B suivantes :
A : 5'-GTGACACTATAGAACGCGGCCGCCA-3' (SEQ ID NO: 18 dans le listage de séquences en annexe) et
B : 5'-TATAGGGAGACCGGCAGATCCGCGG-3' (SEQ ID NO: 19 dans le listage de séquences en annexe).

Ces amorces peuvent contenir des séquences d'homologie aux séquences proches du locus cible. Cette homologie permet l'intégration site spécifique des séquences ParS en utilisant la recombinaison homologue chez la levure.

Des vecteurs contenant des sites Lox, flanquant la région qui contient le marqueur de sélection, peuvent être utilisés pour récupérer l'auxotrophie pour le marqueur de sélection et ainsi réduire la taille de l'insertion au minimum.

ParB-c2-mCherry, ParB-c3-GFP, ParS-c2 et ParS-c3 sont associés avec les marqueurs *URA3, TRP1, NAT* et *HYGRO,* respectivement. De fait, ces quatre constructions peuvent être utilisées simultanément.

### C. Pour utilisation dans des cellules de mammifères.

Pour les cellules de mammifères, les deux constructions ParB-c2-mCherry et ParB-c3-GFP sont sous le contrôle du promoteur CMV présent dans le squelette du plasmide peGFP-c2 (Figure 4). De ce fait, l'expression de ParB est dépendante du temps. Dans le cas d'un double marquage, peGFP-c2 ParB-c2-mCherry et ParB-c3-GFP doivent être transfectés de façon transitoire.

Les séquences ParS sont amplifiées pour l'intégration par PCR en utilisant les couples d'amorces C et D suivantes :.
C : 5'-TCCAGCCCTCACTCCTTCTCTAGGCGCCGGAA-3' (SEQ ID NO: 20 dans le listage de séquences en annexe) et
D : 5'-GTTCTCCAACTTCAAGAAACTGTTACCCAT-3' (SEQ ID NO: 21 dans le listage de séquences en annexe).

Les produits de PCR sont ensuite transfectés dans les cellules et les clones résistant à l'hygromycine sont sélectionnés (Figure 5). Les sites d'insertion peuvent ensuite être déterminés en utilisant des techniques publiées (Ozawa et al, 2004).

### II.2. Détection de séquences ParS in vivo chez les levures et les cellules humaines.

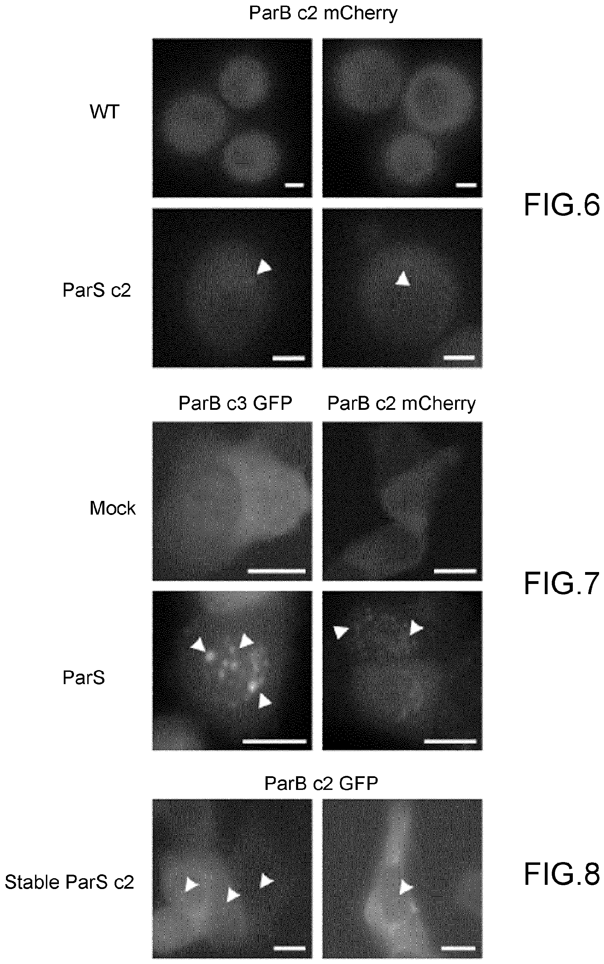

### A. Dans les cellules de levure (Figure 6).

Dans des cellules sauvages (WT) non étiquetées, un signal fluorescent diffus est visualisé dans toute la cellule mise à part la vacuole.

Quand la construction ParS-c2 est insérée dans le génome (ici, le locus *HML*), un point fluorescent brillant induit par la liaison de ParB sur ParS peut être visualisé (tête de flèche). Barres: 2 µm.

### B. Dans les cellules de mammifères avec un ParS plasmidique (Figure 7) .

Les cellules humaines HeLa exprimant de façon transitoire soit la construction peGFP-c2 ParB-c3-GFP ou peGFP-c2 ParB-c2-mCherry sont co-transfectées avec le plasmide qui contient les séquences ParS-c2 ou ParS-c3 (ParS). Dans ce cas, des foci fluorescents multiples peuvent être visualisées, concomitant avec le fait qu'un nombre important de molécules d'ADN plasmidiques entrent dans les cellules lors de la transfection (Batard et al, 2001).

Aucun focus de fluorescence n'est visible dans les cellules humaines HeLa exprimant de façon transitoire soit la construction peGFP-c2 ParB-c3-GFP, soit la construction ou peGFP-c2 ParB-c2-mCherry qui ont été co-transfectées avec de l'eau (Mock). Barres 15 µm.

### C. Dans les cellules de mammifères avec un ParS intégré de façon stable (Figure 8).

Dans une lignée de cellules HeLa ayant stablement intégré une construction ParS-c2 unique et exprimant de façon transitoire la construction peGFP-c2 ParB-c2-GFP, un focus de fluorescence est observé (tête de flèche).

### II.3. Analyse de la dynamique du locus HML.

Cette analyse est conduite en utilisant le système de localisation ParS-c2/ParB-c2-mCherry. La séquence ParS-c2 est intégrée dans le locus *HML* et les cellules expriment la construction pCM189 ParB-c2-mCherry.

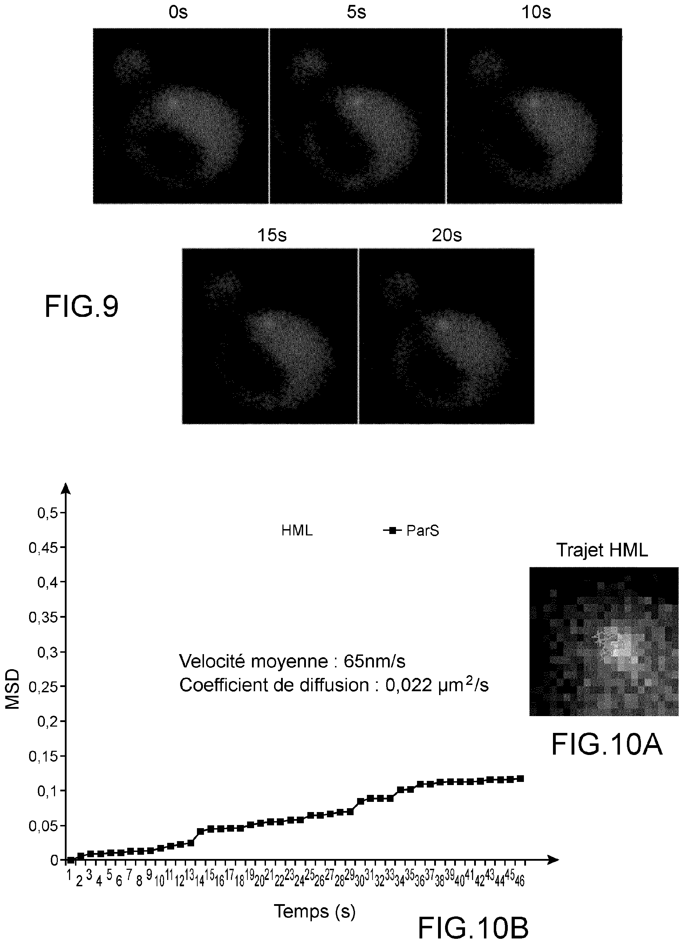

La position du focus mCherry correspond donc à la position du locus HML *in vivo.* Les images ont été prises à un intervalle de 1 sec pendant 50 sec (Figure 9) .

Les piles d'images issues des acquisitions ont été importées dans le logiciel Image J (http://rsbweb.nih.gov/ij/) et la position du focus *HML* a été suivie automatiquement au cours du temps.

Le trajet de *HML* (en clair) durant l'acquisition est présenté Figure 10A. La Figure 10B montre la caractérisation du mouvement de *HML* par analyse de *Mean-Square displacement* (MSD). *HML* adopte un comportement diffusif lent. Les vitesses moyennes ainsi que les coefficients de diffusion calculés sont du même ordre de grandeur que les valeurs précédemment publiées (Bystricky et al, 2005; Bystricky et al, 2009) .

### II.4. Dégradation de la séquence ParS lors de l'induction d'une cassure double brin unique.

Des cellules ayant intégré la construction ParS-c2 au locus *MAT,* à environ 100 nucléotides du site de coupure correspondant à une cassure double-brin unique par l'endonucléase HO, expriment la construction pCM189 ParB-c2-mCherry.

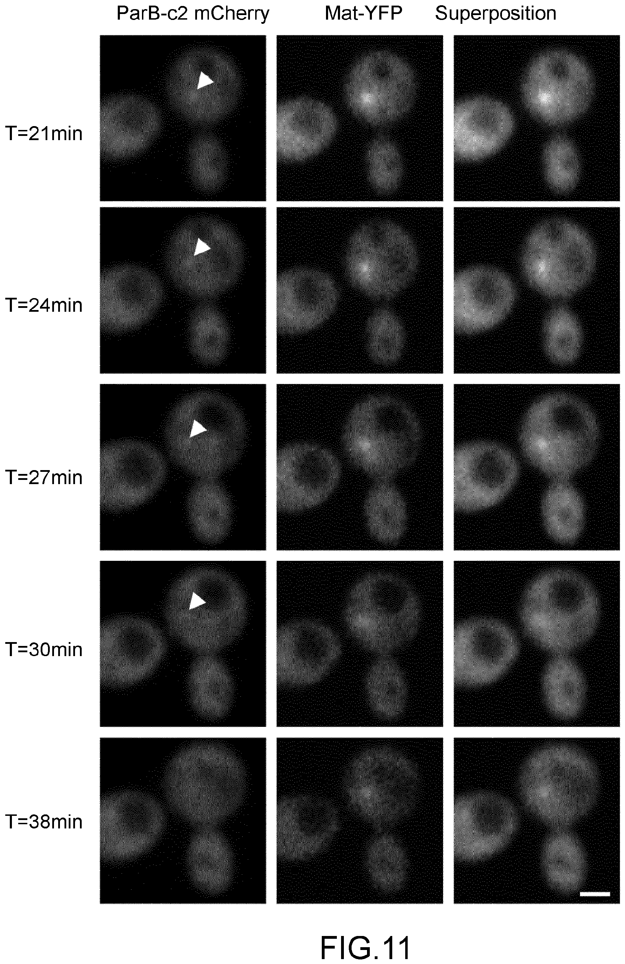

Les cellules sont mises en culture dans du milieu sélectif contenant du raffinose et l'induction de l'endonucléase HO est réalisée par ajout de galactose (pGal-Ho). Les cellules sont imagées en multicouleur sous un microscope confocal rapide ANDOR Revolution équipé d'une tête YOKOGAWA CSU22 et d'une caméra Andor iXon_{EM}+DU888. Les images sont prises avec un objectif Olympus PlanSApo 100x 1,40. La position du locus *MAT* portant une étiquette lacO à 4 kb de distance (a 197 kb du télomère gauche du chromosome 3) est suivie simultanément (Figure 11).

La disparition du foci ParS correspond à la dégradation des séquences du locus *MAT* à proximité du site de coupure HO.

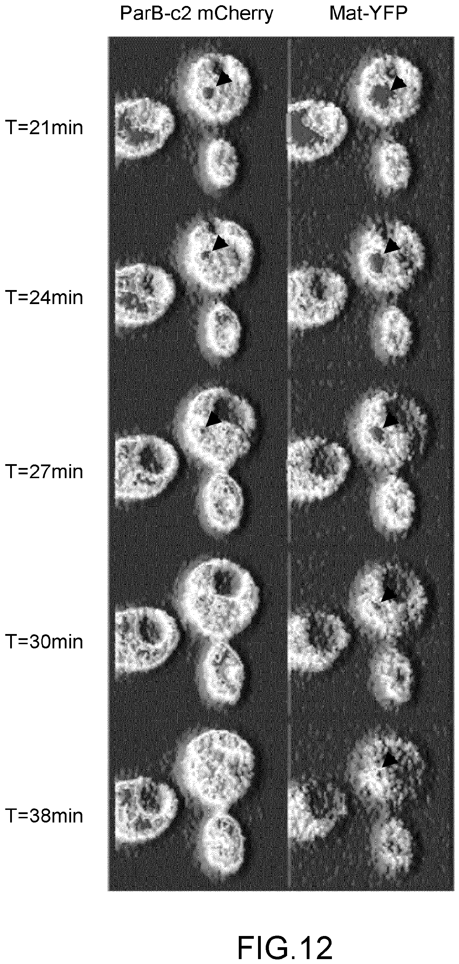

Les images ont été importées dans le programme Image J et la fluorescence, transformée en unité arbitraire, est représentée en 3 dimensions (Figure 12). La position des foci YFP et mCherry est indiquée par des têtes de flêches noires.

La disparition du focus mCherry est visible à partir de 30 min. La perte du focus est spécifique puisque les niveaux de fluorescence totaux mCherry sont sensiblement identiques au cours de l'acquisition, notamment dans le bourgeon.

### RÉFÉRENCES

Batard P, Jordan M, Wurm F (2001) Transfer of high copy number plasmid into mammalian cells by calcium phosphate transfection. Gene 270(1-2): 61-68
Belli G, Gari E, Piedrafita L, Aldea M, Herrero E (1998) An activator/repressor dual system allows tight tetracycline-regulated gene expression in budding yeast. Nucleic Acids Res 26(4): 942-947
Belmont AS (2001) Visualizing chromosome dynamics with GFP. Trends Cell Biol 11(6): 250-257
Belmont AS, Straight AF (1998) In vivo visualization of chromosomes using lac operator-repressor binding. Trends Cell Biol 8(3): 121-124
Bystricky K, Laroche T, van Houwe G, Blaszczyk M, Gasser SM (2005) Chromosome looping in yeast: telomere pairing and coordinated movement reflect anchoring efficiency and territorial organization. J Cell Biol 168(3): 375-387
Bystricky K, Van Attikum H, Montiel MD, Dion V, Gehlen L, Gasser SM (2009) Regulation of nuclear positioning and dynamics of the silent mating type loci by the yeast Ku70/Ku80 complex. Mol Cell Biol 29(3): 835-848
Dubarry N, Pasta F, Lane D (2006) ParABS systems of the four replicons of Burkholderia cenocepacia: new chromosome centromeres confer partition specificity. J Bacteriol 188(4): 1489-1496
Gari E, Piedrafita L, Aldea M, Herrero E (1997) A set of vectors with a tetracycline-regulatable promoter system for modulated gene expression in Saccharomyces cerevisiae. Yeast 13(9): 837-848
Gietz RD, Schiestl RH (1991) Applications of high efficiency lithium acetate transformation of intact yeast cells using single-stranded nucleic acids as carrier. Yeast 7(3): 253-263
Golovanov AP, Barilla D, Golovanova M, Hayes F, Lian LY (2003) ParG, a protein required for active partition of bacterial plasmids, has a dimeric ribbon-helix-helix structure. Mol. Microbiol. 50: 1141-1153.
Li Y, Austin S (2002) The P1 plasmid is segregated to daughter cells by a 'capture and ejection' mechanism coordinated with Escherichia coli cell division. Mol Microbiol 46(1): 63-74
Lin DC, Grossman AD (1998) Identification and characterization of a bacterial chromosome partitioning site. Cell 92(5): 675-685
Livny J, Yamaichi Y, Waldor MK (2007) Distribution of centromere-like parS sites in bacteria: insights from comparative genomics. J Bacteriol 189(23): 8693-8703
Lynch AS, Wang JC (1995) sopB protein-mediated silencing of genes linked to the spoC locus of Escherichia coli F plasmid, Proc Natl Acad Sci U S A 92 (6): 1896-1900
Michaelis C, Ciosk R, Nasmyth K (1997) Cohesins: chromosomal proteins that prevent premature separation of sister chromatids. Cell 91(1): 35-45
Murray H, Ferreira H, Errington J (2006) The bacterial chromosome segregation protein Spo0J spreads along DNA from parS nucleation sites. Mol Microbiol 61(5): 1352-1361
Ozawa T, Itoyama T, Sadamori N, Yamada Y, Hata T, Tomonaga M, Isobe M (2004) Rapid isolation of viral integration site reveals frequent integration of HTLV-1 into expressed loci. J Hum Genet 49(3): 154-165
Straight AF, Belmont AS, Robinett CC, Murray AW (1996) GFP tagging of budding yeast chromosomes reveals that protein-protein interactions can mediate sister chromatid cohesion. Curr Biol 6(12): 1599-1608
Surtees JA, Funnell BE (1999) P1 ParB domain structure includes two independent multimerization domains. J. Bacteriol 181(19): 5898-5908
Therizols P, Duong T, Dujon B, Zimmer C, Fabre E Chromosome arm length and nuclear constraints determine the dynamic relationship of yeast subtelomeres. Proc Natl Acad Sci U S A 107(5): 2025-2030

## Revendications

1. Utilisation non thérapeutique d'un vecteur d'expression eucaryote pour contrôler l'expression d'un gène *in vivo* chez un eucaryote à l'exception d'un humain ou pour détecter la position, la dynamique et la dégradation de loci d'ADN ou la recombinaison ou l'échange de séquences d'ADN *in vivo* chez un eucaryote,
ledit vecteur d'expression eucaryote comprenant une séquence nucléotidique codant une protéine fusion comprenant
un 1^{er} polypeptide correspondant à une protéine liant l'ADN, sans qu'un quelconque autre facteur ne soit nécessaire à cette liaison, et appartenant au système de partition de l'ADN bactérien, un dérivé ou un fragment de celle-ci et
un 2^{nd} polypeptide qui est soit un polypeptide facilement détectable, soit un polypeptide impliqué dans la régulation de l'expression des gènes.

2. Utilisation non thérapeutique d'un vecteur selon la revendication 1, **caractérisée en ce que** la protéine liant l'ADN et appartenant au système de partition de l'ADN bactérien est une protéine ParB issue de *Burkholderia cenocepacia.*

3. Utilisation non thérapeutique d'un vecteur selon la revendication 1 ou 2, **caractérisée en ce que** la protéine liant l'ADN et appartenant au système de partition de l'ADN bactérien est choisie entre :
- la protéine ParB codée par le chromosome 1 de *Bcc* désignée par « ParB-c1 » telle que la protéine de la souche J2315 de *Bcc* accessible dans NCBI genome project sous le N° YP-002229191 ;
- la protéine ParB codée par le chromosome 2 de *Bcc* désignée par « ParB-c2 » telle que la protéine de la souche J2315 de *Bcc* accessible dans NCBI genome project sous le N° YP-002232636 et correspondant à la protéine SEQ ID NO: 2 dans le listage de séquences en annexe ;
- la protéine ParB codée par le chromosome 3 de *Bcc* désignée par « ParB-c3 » telle que la protéine de la souche J2315 de *Bcc* accessible dans NCBI genome project sous le N° YP-002153395 et correspondant à la protéine SEQ ID NO: 4 dans le listage de séquences en annexe ; et
- la protéine ParB codée par le plasmide de *Bcc* désignée par « ParB-pl » telle que la protéine du plasmide pBCJ2315 de la souche J2315 de *Bcc* accessible dans NCBI genome project sous le N° YP-002235439,
un dérivé ou un fragment de celles-ci.

4. Utilisation non thérapeutique d'un vecteur selon l'une quelconque des revendications 1 à 3, **caractérisée en ce que** ledit polypeptide facilement détectable est
- une enzyme capable de générer un signal détectable et éventuellement quantifiable dans des conditions particulières comme lors de la mise en présence d'un substrat adapté ou
- un polypeptide bioluminescent ou fluorescent.

5. Utilisation non thérapeutique d'un vecteur selon l'une quelconque des revendications 1 à 3, **caractérisée en ce que** ledit polypeptide impliqué dans la régulation de l'expression des gènes est choisi parmi un facteur de transcription positif ou négatif, un facteur de transcription général, un régulateur de l'expression d'un promoteur, un facteur de remodelage de la chromatine et un facteur modifiant la localisation d'un gène adjacent, un dérivé ou un fragment de ceux-ci.

6. Utilisation non thérapeutique d'un vecteur selon l'une quelconque des revendications précédentes, **caractérisée en ce qu'**il comprend, liés entre eux de façon opérationnelle, un promoteur de type eucaryote, une séquence nucléotidique codant une protéine de fusion et un signal de terminaison de la transcription eucaryote comprenant un site de clivage et/ou un signal polyA.

7. Utilisation non thérapeutique d'une protéine de fusion telle que définie à l'une quelconque des revendications 1 à 5 pour contrôler l'expression d'un gène *in vivo* chez un eucaryote à l'exception d'un humain ou pour détecter la position, la dynamique et la dégradation de loci d'ADN ou la recombinaison ou l'échange de séquences d'ADN *in vivo* chez un eucaryote.

8. Utilisation non thérapeutique d'un vecteur eucaryote pour contrôler l'expression d'un gène *in vivo* chez un eucaryote à l'exception d'un humain ou pour détecter la position, la dynamique et la dégradation de loci d'ADN ou la recombinaison ou l'échange de séquences d'ADN *in vivo* chez un eucaryote,
ledit vecteur eucaryote comprenant une séquence nucléotidique présentant au moins un site de reconnaissance par une protéine liant l'ADN et appartenant au système de partition de l'ADN bactérien et un marqueur de sélection de type eucaryote.

9. Utilisation non thérapeutique d'un vecteur selon la revendication 8, ledit vecteur étant intégratif.

10. Utilisation non thérapeutique d'un vecteur selon la revendication 8 ou 9, **caractérisée en ce que** ledit site de reconnaissance par une protéine liant l'ADN et appartenant au système de partition de l'ADN bactérien est de séquence nucléotidique (I) ou de séquence complémentaire à la séquence nucléotidique (I) :
N₁N₂TN₃N₄N₅N₆CGN₇N₈N₉N₁₀AN₁₁N₁₂ (I) (SEQ ID NO: 5 dans le listage de séquences en annexe) dans laquelle les nucléotides N₁ et N₁₂, identiques ou différents, sont choisis parmi A, G, C ou T et les couples de nucléotides (N₆,N₇), (N₅,N₈), (N₄,N₉), (N₃,N₁₀) et (N₂,N₁₁) indépendamment les uns des autres sont choisis dans le groupe constitué par (A,T), (T,A), (C,G) et (G,C), les nucléotides N₁ et N₁₂ pouvant éventuellement être absents.

11. Utilisation non thérapeutique d'un vecteur selon l'une quelconque des revendications 8 à 10, **caractérisée en ce que** ledit site de reconnaissance par une protéine liant l'ADN et appartenant au système de partition de l'ADN bactérien présente une séquence nucléotidique choisie parmi les séquences nucléotidiques suivantes :
- GTTTATGCGCATAAAC (SEQ ID NO: 7 dans le listage de séquences en annexe) ;
- CTTTATGCGCATAAAC (SEQ ID NO: 8 dans le listage de séquences en annexe) ;
- GTTGTCACGTGACAAC (SEQ ID NO: 9 dans le listage de séquences en annexe) ;
- TTTGTCACGTGACAAC (SEQ ID NO: 10 dans le listage de séquences en annexe) ;
- CTTGTCACGTGACAAC (SEQ ID NO: 11 dans le listage de séquences en annexe) ;
et une séquence complémentaire à l'une quelconque de ces séquences.

12. Utilisation non thérapeutique d'un vecteur selon la revendication 8 à 11, **caractérisée en ce que** ladite séquence nucléotidique comprend au moins 2, au moins 3 ou au moins 4 sites de reconnaissance par une protéine liant l'ADN et appartenant au système de partition de l'ADN bactérien.

13. Utilisation non thérapeutique d'un vecteur selon la revendication 8 à 12, **caractérisée en ce que** ladite séquence nucléotidique correspond à une séquence choisie parmi :
- la séquence ParS portée par le chromosome 1 de *Bcc* désignée par « ParS-c1 » qui peut être obtenue à partir de la séquence nucléotidique du chromosome 1 de la souche J2315 de *Bcc* accessible dans Genbank sous le N° AM 747720, les deux sites de reconnaissance que contient cette séquence se trouvant respectivement aux nucléotides 26477-26492 et 28403-28418 ;
- la séquence ParS portée par le chromosome 2 de *Bcc* désignée par « ParS-c2 » qui peut être obtenue à partir de la séquence nucléotidique du chromosome 2 de la souche J2315 de *Bcc* accessible dans Genbank sous le N° AM 747721 et qui correspondant à la protéine SEQ ID NO: 12 dans le listage de séquences en annexe ;
- la séquence ParS portée par le chromosome 3 de *Bcc* désignée par « ParS-c3 » qui peut être obtenue à partir de la séquence nucléotidique du chromosome 3 de la souche J2315 de *Bcc* accessible dans Genbank sous le N° AM 747722 et qui correspondant à la protéine SEQ ID NO: 13 dans le listage de séquences en annexe ;
- la séquence ParS portée par le plasmide de *Bcc* désignée par « ParS-p1 » qui peut être obtenue à partir de la séquence nucléotidique du plasmide pBCJ2315 de la souche J2315 de *Bcc* accessible dans Genbank sous le N° AM 747723, les trois sites de reconnaissance que contient cette séquence se trouvant respectivement aux nucléotides 92285-92300, 92385-92400 et 92438-92453 ;
ou une séquence complémentaire à l'une quelconque de ces séquences.

14. Utilisation non thérapeutique d'un organisme hôte eucaryote pour contrôler l'expression d'un gène *in vivo* chez un eucaryote ou pour détecter la position, la dynamique et la dégradation de loci d'ADN ou la recombinaison ou l'échange de séquences d'ADN *in vivo* chez un eucaryote,
ledit organisme hôte eucaryote étant, transformé par ou comprenant un vecteur d'expression eucaryote tel que défini à l'une quelconque des revendications 1 à 6 ou un vecteur eucaryote tel que défini à l'une quelconque des revendications 8 à 13,
ledit organisme hôte eucaryote n'étant pas un humain.

15. Utilisation non thérapeutique d'un organisme hôte eucaryote selon la revendication 14, **caractérisée en ce que** ledit organisme est un micro-organisme tel qu'une levure ou un champignon ; une cellule animale telle qu'une cellule d'insecte ou une cellule de mammifère et notamment une cellule humaine, une cellule de hamster, une cellule de singe, une cellule de lapin, une cellule de souris, une cellule de rat ; une cellule végétale ; une plante ; ou un animal à l'exception d'un humain.

16. Utilisation non thérapeutique d'un organisme hôte eucaryote selon la revendication 14 ou 15, **caractérisée en ce que** ledit organisme a intégré dans son génome la séquence nucléotidique présentant au moins un site de reconnaissance par une protéine liant l'ADN et appartenant au système de partition de l'ADN bactérien et exprime une protéine de fusion telle que définie à l'une quelconque des revendications 1 à 5.

17. Utilisation d'au moins un élément choisi parmi :
- un vecteur d'expression eucaryote selon l'une quelconque des revendications 1 à 6 ;
- une protéine de fusion telle que définie à l'une quelconque des revendications 1 à 5 ;
- une séquence nucléotidique telle que définie à l'une quelconque des revendications 8 ou 10 à 13 ;
- un vecteur eucaryote tel que défini à l'une quelconque des revendications 8 à 13 ; et
- un organisme hôte tel que défini à l'une quelconque des revendications 14 à 16,
pour détecter une molécule susceptible de contrôler l'expression d'un gène *in vivo* chez un eucaryote ou susceptible d'affecter la position, la dynamique et la dégradation de loci d'ADN ou la recombinaison ou l'échange de séquences d'ADN *in vivo* chez un eucaryote.

18. Elément choisi parmi :
- un vecteur d'expression eucaryote selon l'une quelconque des revendications 1 à 3, 5 et 6 ;
- une protéine de fusion telle que définie la revendication 5 ;
- une séquence nucléotidique telle que définie à l'une quelconque des revendications 8 ou 10 à 13 ;
- un vecteur eucaryote tel que défini à l'une quelconque des revendications 8 à 13 ; et
- un organisme hôte tel que défini à l'une quelconque des revendications 14 à 16,
pour utilisation dans le traitement d'un cancer ou d'une maladie génétique.

## Patentansprüche

1. Nicht-therapeutische Verwendung eines eukaryotischen Expressionsvektors zum Steuern der Expression eines Gens *in vivo* bei einem Eukaryoten, ausgenommen einem Menschen, oder zum Erfassen der Position, der Dynamik und des Abbaus von DNA-Loci oder der Rekombination oder des Austauschs von DNA-Sequenzen *in vivo* bei einem Eukaryoten,
wobei der eukaryotische Expressionsvektor eine Nukleotidsequenz umfasst, die ein Fusionsprotein kodiert, umfassend
ein 1. Polypeptid, das einem DNA-bindenden Protein entspricht, bei dem für die Bindung keinerlei weiterer Faktor erforderlich ist und das zum Teilungssystem von bakterieller DNA gehört, ein Derivat oder ein Fragment davon, und
ein 2. Polypeptid, bei dem es sich um ein leicht nachweisbares Polypeptid handelt, und das ein Polypeptid ist, das an der Regulation der Expression von Genen beteiligt ist.

2. Nicht-therapeutische Verwendung eines Vektors nach Anspruch 1, **dadurch gekennzeichnet, dass** das DNA-bindende Protein, das zum Teilungssystem von bakterieller DNA gehört, ein Protein ParB ist, das von *Burkholderia cenocepacia* erzeugt wird.

3. Nicht-therapeutische Verwendung eines Vektors nach Anspruch 1 oder 2, **dadurch gekennzeichnet, dass** das DNA-bindende Protein, das zum Teilungssystem von bakterieller DNA gehört, ausgewählt ist aus:
- dem Protein ParB, das durch das Chromosom 1 von *Bcc,* bezeichnet als « ParB-c1 », kodiert wird, wie z.B. das Protein des Stamms J2315 von *Bcc,* das in dem NCBI-Genomprojekt unter der Nr. YP-002229191 zugänglich ist;
- dem Protein ParB, das durch das Chromosom 2 von *Bcc,* bezeichnet als « ParB-c2 », kodiert wird, wie z.B. das Protein des Stamms J2315 von *Bcc,* das in dem NCBI-Genomprojekt unter der Nr. YP-002232636 zugänglich ist und dem Protein SEQ ID NO: 2 in dem beigefügten Sequenzprotokoll entspricht;
- dem Protein ParB, das durch das Chromosom 3 von *Bcc,* bezeichnet als « ParB-c3 », kodiert wird, wie z.B. das Protein des Stamms J2315 von *Bcc,* das in dem NCBI-Genomprojekt unter der Nr. YP-002153395 zugänglich ist und dem Protein SEQ ID NO: 4 in dem beigefügten Sequenzprotokoll entspricht; und
- dem Protein ParB, das durch das Plasmid von *Bcc,* bezeichnet als « ParB-p1 », kodiert wird, wie z.B. das Protein des Plasmids pBCJ2315 des Stamms J2315 von *Bcc,* das in dem NCBI-Genomprojekt unter der Nr. YP-002235439 zugänglich ist;
einem Derivat oder einem Fragment davon.

4. Nicht-therapeutische Verwendung eines Vektors nach einem der Ansprüche 1 bis 3, **dadurch gekennzeichnet, dass** das leicht nachweisbare Polypeptid
- ein Enzym, das ein Signal erzeugen kann, das gegebenenfalls unter speziellen Bedingungen quantifizierbar ist, wie wenn es in ein angepasstes Substrat eingebracht wird, oder
- ein biolumineszierendes oder fluoreszierendes Polypeptid ist.

5. Nicht-therapeutische Verwendung eines Vektors nach einem der Ansprüche 1 bis 3, **dadurch gekennzeichnet, dass** das Polypeptid, das an der Regulierung der Expression von Genen beteiligt ist, aus einem positiven oder negativen Transkriptionsfaktor, einem allgemeinen Transkriptionsfaktor, einem Regulator der Expression eines Promotors, einem Chromatin-Umgestaltungsfaktor und einem Faktor, der die Position eines benachbarten Gens modifiziert, einem Derivat oder einem Fragment davon ausgewählt ist.

6. Nicht-therapeutische Verwendung eines Vektors nach einem der vorhergehenden Ansprüche, **dadurch gekennzeichnet, dass** er in einer dazwischen liegenden funktionellen Weise einen Promotor des eukaryotischen Typs, eine Nukleotidsequenz, die ein Fusionsprotein kodiert, und ein Terminationssignal der eukaryotischen Transkription umfasst, das eine Spaltungsstelle und/oder ein polyA-Signal umfasst.

7. Nicht-therapeutische Verwendung eines Fusionsproteins nach einem der Ansprüche 1 bis 5 zum Steuern der Expression eines Gens *in vivo* bei einem Eukaryoten, ausgenommen einem Menschen, oder zum Erfassen der Position, der Dynamik und des Abbaus von DNA-Loci oder der Rekombination oder des Austauschs von DNA-Sequenzen *in vivo* bei einem Eukaryoten.

8. Nicht-therapeutische Verwendung eines eukaryotischen Vektors zum Steuern der Expression eines Gens *in vivo* bei einem Eukaryoten, ausgenommen einem Menschen, oder zum Erfassen der Position, der Dynamik und des Abbaus von DNA-Loci oder der Rekombination oder des Austauschs von DNA-Sequenzen *in vivo* bei einem Eukaryoten,
wobei der eukaryotische Expressionsvektor eine Nukleotidsequenz, die mindestens eine Erkennungsstelle für ein DNA-bindendes Protein präsentiert, das zum Teilungssystem von bakterieller DNA gehört, und einen Selektionsmarker des eukaryotischen Typs umfasst.

9. Nicht-therapeutische Verwendung eines Vektors nach Anspruch 8, wobei der Vektor integrierend ist.

10. Nicht-therapeutische Verwendung eines Vektors nach Anspruch 8 oder 9, **dadurch gekennzeichnet, dass** die Erkennungsstelle für ein DNA-bindendes Protein, das zum Teilungssystem von bakterieller DNA gehört, die Nukleotidsequenz (I) oder die zu der Nukleotidsequenz (I) komplementäre Sequenz ist:
N₁N₂TN₃N₄N₅N₆CGN₇N₈N₉N₁₀AN₁₁N₁₂ (I) (SEQ ID NO: 5 in dem beigefügten Sequenzprotokoll), bei der die Nukleotide N₁ und N₁₂, die identisch oder verschieden sind, ausgewählt sind aus A, G, C oder T, und die Paare der Nukleotide (N₆, N₇), (N₅, N₈), (N₄, N₉), (N₃, N₁₀) und (N₂, N₁₁) unabhängig voneinander ausgewählt sind aus der Gruppe, bestehend aus (A, T), (T, A), (C, G) und (G, C), wobei die Nukleotide N₁ und N₁₂ gegebenenfalls nicht vorliegen.

11. Nicht-therapeutische Verwendung eines Vektors nach einem der Ansprüche 8 bis 10, **dadurch gekennzeichnet, dass** die Erkennungsstelle für ein DNA-bindendes Protein, das zum Teilungssystem von bakterieller DNA gehört, eine Nukleotidsequenz aufweist, die aus den nachstehenden Nukleotidsequenzen ausgewählt ist:
- GTTTATGCGCATAAAC (SEQ ID NO: 7 in dem beigefügten Sequenzprotokoll);
- CTTTATGCGCATAAAC (SEQ ID NO: 8 in dem beigefügten Sequenzprotokoll);
- GTTGTCACGTGACAAC (SEQ ID NO: 9 in dem beigefügten Sequenzprotokoll);
- TTTGTCACGTGACAAC (SEQ ID NO: 10 in dem beigefügten Sequenzprotokoll);
- CTTGTCACGTGACAAC (SEQ ID NO: 11 in dem beigefügten Sequenzprotokoll);
und einer Sequenz, die zu einer dieser Sequenzen komplementär ist.

12. Nicht-therapeutische Verwendung eines Vektors nach einem der Ansprüche 8 bis 11, **dadurch gekennzeichnet, dass** die Nukleotidsequenz mindestens 2, mindestens 3 oder mindestens 4 Erkennungsstellen für ein DNA-bindendes Protein, das zum Teilungssystem von bakterieller DNA gehört, umfasst.

13. Nicht-therapeutische Verwendung eines Vektors nach einem der Ansprüche 8 bis 12, **dadurch gekennzeichnet, dass** die Nukleotidsequenz einer Sequenz entspricht, die ausgewählt ist aus:
- der Sequenz ParS, die auf dem Chromosom 1 von *Bcc,* bezeichnet als « ParS-c1 », vorliegt, die von der Nukleotidsequenz von Chromosom 1 des Stamms J2315 von *Bcc* erhältlich ist, das in der Genbank unter der Nr. AM 747720 zugänglich ist, wobei die zwei Erkennungsstellen, welche diese Sequenz enthalten, bei den Nukleotiden 26477-26492 bzw. 28403-28418 vorliegen;
- der Sequenz ParS, die auf dem Chromosom 2 von *Bcc,* bezeichnet als « ParS-c2 », vorliegt, die von der Nukleotidsequenz von Chromosom 2 des Stamms J2315 von *Bcc* erhältlich ist, das in der Genbank unter der Nr. AM 747721 zugänglich ist und die dem Protein SEQ ID NO: 12 in dem beigefügten Sequenzprotokoll entspricht;
- der Sequenz ParS, die auf dem Chromosom 3 von *Bcc,* bezeichnet als « ParS-c3 », vorliegt, die von der Nukleotidsequenz von Chromosom 3 des Stamms J2315 von *Bcc* erhältlich ist, das in der Genbank unter der Nr. AM 747722 zugänglich ist und die dem Protein SEQ ID NO: 13 in dem beigefügten Sequenzprotokoll entspricht;
- der Sequenz ParS, die in dem Plasmid von *Bcc,* bezeichnet als « ParS-p1 », vorliegt, die von der Nukleotidsequenz des Plasmids pBCJ2315 des Stamms J2315 von *Bcc* erhältlich ist, das in der Genbank unter der Nr. AM 747723 zugänglich ist, wobei die drei Erkennungsstellen, welche diese Sequenz enthalten, bei den Nukleotiden 92285-92300, 92385-92400 bzw. 92438-92453 vorliegen;
oder einer Sequenz, die zu einer dieser Sequenzen komplementär ist.

14. Nicht-therapeutische Verwendung eines eukaryotischen Wirtsorganismus zum Steuern der Expression eines Gens *in vivo* bei einem Eukaryoten oder zum Erfassen der Position, der Dynamik und des Abbaus von DNA-Loci oder der Rekombination oder des Austauschs von DNA-Sequenzen *in vivo* bei einem Eukaryoten,
wobei der eukaryotische Wirtsorganismus ein eukaryotischer Expressionsvektor nach einem der Ansprüche 1 bis 6 oder ein eukaryotischer Vektor nach einem der Ansprüche 8 bis 13 ist, durch diesen transformiert worden ist oder diesen umfasst,
wobei der eukaryotische Wirtsorganismus kein Mensch ist.

15. Nicht-therapeutische Verwendung eines eukaryotischen Wirtsorganismus nach Anspruch 14, **dadurch gekennzeichnet, dass** der Organismus ein Mikroorganismus, wie z.B. eine Hefe oder ein Pilz; eine tierische Zelle, wie z.B. eine Insektenzelle oder eine Säugerzelle und insbesondere eine menschliche Zelle, eine Hamsterzelle, eine Affenzelle, eine Kaninchenzelle, eine Mauszelle, eine Rattenzelle; eine Pflanzenzelle; eine Pflanze; oder ein Tier, ausgenommen ein Mensch, ist.

16. Nicht-therapeutische Verwendung eines eukaryotischen Wirtsorganismus nach Anspruch 14 oder 15, **dadurch gekennzeichnet, dass** der Organismus in seinem Genom die Nukleotidsequenz integriert hat, die mindestens eine Erkennungsstelle für ein DNA-bindendes Protein präsentiert, das zum Teilungssystem von bakterieller DNA gehört, und ein Fusionsprotein nach einem der Ansprüche 1 bis 5 exprimiert.

17. Verwendung mindestens eines Elements, ausgewählt aus:
- einem eukaryotischen Expressionsvektor nach einem der Ansprüche 1 bis 6;
- einem Fusionsprotein nach einem der Ansprüche 1 bis 5;
- einer Nukleotidsequenz nach einem der Ansprüche 8 oder 10 bis 13;
- einem eukaryotischen Vektor nach einem der Ansprüche 8 bis 13; und
- einem Wirtsorganismus nach einem der Ansprüche 14 bis 16;
zum Nachweisen eines Moleküls, das einer Steuerung der Expression durch ein Gen in vivo bei einem Eukaryoten unterliegt, oder das die Position, die Dynamik und den Abbau von DNA-Loci oder die Rekombination oder den Austausch von DNA-Sequenzen *in vivo* bei einem Eukaryoten beeinflusst.

18. Element, ausgewählt aus:
- einem eukaryotischen Expressionsvektor nach einem der Ansprüche 1 bis 3, 5 und 6;
- einem Fusionsprotein nach Anspruch 5;
- einer Nukleotidsequenz nach einem der Ansprüche 8 oder 10 bis 13;
- einem eukaryotischen Vektor nach einem der Ansprüche 8 bis 13; und
- einem Wirtsorganismus nach einem der Ansprüche 14 bis 16;
zur Verwendung bei der Behandlung von Krebs oder einer genetischen Erkrankung.

## Claims

1. Non-therapeutic use of a eukaryotic expression vector to control the expression of a gene *in vivo* in a eukaryote with the exception of a human or to detect the position, the dynamics and the degradation of DNA loci or the recombination or exchange of DNA sequences *in vivo* in a eukaryote,
said expression vector comprising a nucleotide sequence encoding a fusion protein comprising
a 1^{st} polypeptide corresponding to a DNA binding protein, without any other factor being necessary for this binding, and which belongs to the partitioning system of bacterial DNA, a derivative or fragment thereof and
a 2^{nd} polypeptide which is either an easily detectable polypeptide or a polypeptide involved in the regulation of gene expression.

2. Non-therapeutic use of a vector according to claim 1, **characterized in that** the DNA binding protein belonging to the partitioning system of bacterial DNA is a ParB protein derived from *Burkholderia cenocepacia.*

3. Non-therapeutic use of a vector according to claim 1 or 2, **characterized in that** the DNA binding protein belonging to the partitioning system of bacterial DNA is chosen between:
- the ParB protein encoded by chromosome 1 of *Bcc* designated « ParB-c1 » such as the protein of *Bcc* strain J2315 accessible in NCBI genome project under N° YP-002229191;
- the ParB protein encoded by chromosome 2 of *Bcc* designated « ParB-c2 » such as the protein of *Bcc* strain J2315 accessible in NCBI genome project under N° YP-002232636 and corresponding to the protein SEQ ID NO: 2 in the appended sequence listing;
- the ParB protein encoded by chromosome 3 of *Bcc* designated « ParB-c3 » such as the protein of *Bcc* strain J2315 accessible in NCBI genome project under N° YP-002153395 and corresponding to the protein SEQ ID NO: 4 in the appended sequence listing; and
- the ParB protein encoded by the plasmid of *Bcc* designated « ParB-pl » such as the protein of the pBCJ2315 plasmid of *Bcc* strain J2315 accessible in NCBI genome project under N° YP-002235439,
a derivative or fragment thereof.

4. Non-therapeutic use of a vector according to any of claims 1 to 3, **characterized in that** said easily detectable polypeptide is:
- an enzyme capable of generating a detectable, optionally quantifiable signal under particular conditions such as when placed in the presence of an adapted substrate; or
- a bioluminescent or fluorescent polypeptide.

5. Non-therapeutic use of a vector according to any of claims 1 to 3, **characterized in that** said polypeptide involved in the regulation of gene expression is chosen from among a positive or negative transcription factor, a general transcription factor, a regulator of the expression of a promoter, a chromatin remodelling factor and a factor modifying the location of an adjacent gene, a derivative or fragment thereof.

6. Non-therapeutic use of a vector according to any of the preceding claims, **characterized in that** it comprises, operationally linked, a promoter of eukaryotic type, a nucleotide sequence encoding a fusion protein and a eukaryotic transcription termination signal comprising a cleavage site and/or polyA signal.

7. Non-therapeutic use of a fusion protein such as defined in any of claims 1 to 5 to control the expression of a gene *in vivo* in a eukaryote with the exception of a human or to detect the position, the dynamics and the degradation of DNA loci or the recombination or exchange of DNA sequences *in vivo* in a eukaryote.

8. Non-therapeutic use of a eukaryotic vector to control the expression of a gene *in vivo* in a eukaryote with the exception of a human or to detect the position, the dynamics and the degradation of DNA loci or the recombination or exchange of DNA sequences *in vivo* in a eukaryote,
said eukaryotic vector comprising a nucleotide sequence having at least one recognition site recognised by a DNA binding protein belonging to the partitioning system of bacterial DNA and a selectable marker of eukaryotic type.

9. Non-therapeutic use of a vector according to claim 8, said vector being integrative.

10. Non-therapeutic use of a vector according to claim 8 or 9, **characterized in that** said recognition site recognised by a DNA binding protein belonging to the partitioning system of bacterial DNA is of nucleotide sequence (I) or a sequence complementary to nucleotide sequence (I) :
N₁N₂TN₃N₄N₅N₆CGN₇N₈N₉N₁₀AN₁₁N₁₂ (I) (SEQ ID NO: 5 in the appended sequence listing) wherein the nucleotides N₁ and N₁₂, the same or different, or chosen from among A, G, C or T and the pairs of nucleotides (N₆,N₇), (N₅,N₈), (N₄,N₉), (N₃,N₁₀) and (N₂,N₁₁) independently of each other are chosen from the group formed by (A,T), (T,A), (C,G) et (G,C), the nucleotides N₁ and N₁₂ possibly being optionally absent.

11. Non-therapeutic use of a vector according to any of claims 8 to 10, **characterized in that** said recognition site, recognised by a DNA binding protein belonging to the partitioning system of bacterial DNA, has a nucleotide sequence chosen from among the following nucleotide sequences:
- GTTTATGCGCATAAAC (SEQ ID NO: 7 in the appended sequence listing);
- CTTTATGCGCATAAAC (SEQ ID NO: 8 in the appended sequence listing);
- GTTGTCACGTGACAAC (SEQ ID NO: 9 in the appended sequence listing);
- TTTGTCACGTGACAAC (SEQ ID NO: 10 in the appended sequence listing);
- CTTGTCACGTGACAAC (SEQ ID NO: 11 in the appended sequence listing);
and a sequence complementary to any one of these sequences.

12. Non-therapeutic use of a vector according to any of claims 8 to 11, **characterized in that** said nucleotide sequence comprises at least 2, at least 3 or at least 4 recognition sites recognised by a DNA binding protein belonging to the partitioning system of bacterial DNA.

13. Non-therapeutic use of a vector according to any of claims 8 to 12, **characterized in that** said nucleotide sequence corresponds to a sequence chosen from among:
- the ParS sequence carried by chromosome 1 of *Bcc* designated « ParS-c1 » which can be obtained from the nucleotide sequence of chromosome 1 of *Bcc* strain J2315 accessible in Genbank under N° AM 747720, the two recognition sites contained in this sequence lying at nucleotides 26477-26492 and 28403-28418 respectively;
- the ParS sequence carried by chromosome 2 of *Bcc* designated « ParS-c2 » which can be obtained from the nucleotide sequence of chromosome 2 of *Bcc* strain J2315 accessible in Genbank under N° AM 747721 and which corresponds to protein SEQ ID NO: 12 in the appended sequence listing;
- the ParS sequence carried by chromosome 3 of *Bcc* designated « ParS-c3 » which can be obtained from the nucleotide sequence of chromosome 3 of *Bcc* strain J2315 accessible in Genbank under N° AM 747722 and which corresponds to protein SEQ ID NO: 13 in the appended sequence listing;
- the ParS sequence carried by the plasmid of *Bcc* designated « ParS-p1 » which can be obtained from the nucleotide sequence of plasmid pBCJ2315 of *Bcc* strain J2315 accessible in Genbank under N° AM 747723, the three recognition sites contained in this sequence respectively lying at nucleotides 92285-92300, 92385-92400 and 92438-92453;
or a sequence complementary to any one of these sequences.

14. Non-therapeutic use of a eukaryotic host organism to control the expression of a gene *in vivo* in a eukaryote or to detect the position, the dynamics and the degradation of DNA loci or the recombination or exchange of DNA sequences *in vivo* in a eukaryote,
said eukaryotic host organism being transformed by or comprising a eukaryotic expression vector such as defined in any of claims 1 to 6 or a eukaryotic vector such as defined in any of claims 8 to 13,
with the exception of a human.

15. Non-therapeutic use of a eukaryotic host organism according to claim 14, **characterized in that** said organism is a micro-organism such as a yeast or fungus; an animal cell such as an insect cell or mammalian cell and in particular a human cell, hamster cell, monkey cell, rabbit cell, mouse cell, rat cell; a plant cell; a plant; or an animal with the exception of a human.

16. Non-therapeutic use of a eukaryotic host organism according to claim 14 or 15, **characterized in that** said organism has integrated in its genome the nucleotide sequence having at least one recognition site recognised by a DNA binding protein belonging to the partitioning system of bacterial DNA, and expresses a fusion protein such as defined in any of claims 1 to 5.

17. Use of at least one element chosen from among:
- a eukaryotic expression vector according to any of claims 1 to 6;
- a fusion protein such defined in any of claims 1 to 5;
- a nucleotide sequence such as defined in any of claims 8 or 10 to 13;
- a eukaryotic vector such as defined in any of claims 8 to 13; and
- a host organism such as defined in any of claims 14 to 16,
to detect a molecule able to control the expression of a gene *in vivo* in a eukaryote or able to affect the position, the dynamics and the degradation of DNA loci or the recombination or exchange of DNA sequences *in vivo* in a eukaryote.

18. Element chosen from among:
- a eukaryotic expression vector according to any of claims 1 to 3, 5 and 6;
- a fusion protein such as defined in claim 5;
- a nucleotide sequence such as defined in any of claims 8 or 10 to 13;
- a eukaryotic vector such as defined in any of claims 8 to 13; and
- a host organism such as defined in any of claims 14 to 16,
for use in treating a cancer or a genetic disease.
